(19) Europäisches Patentamt
European Patent Office
Office européen des brevets

(11) EP 4 393 991 A1

(12) EUROPEAN PATENT APPLICATION
published in accordance with Art. 153(4) EPC

(43) Date of publication:
**03.07.2024 Bulletin 2024/27**

(21) Application number: **22887677.7**

(22) Date of filing: **28.10.2022**

(51) International Patent Classification (IPC):
***C08K 5/103*** (2006.01) ***C08L 33/06*** (2006.01)
***C08J 3/24*** (2006.01) ***C08J 3/12*** (2006.01)

(52) Cooperative Patent Classification (CPC):
**C08J 3/12; C08J 3/24; C08K 5/103; C08L 33/06**

(86) International application number:
**PCT/KR2022/016642**

(87) International publication number:
**WO 2023/075482 (04.05.2023 Gazette 2023/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.10.2021 KR 20210147025**
**27.10.2022 KR 20220140642**

(71) Applicant: **LG Chem, Ltd.**
**Yeongdeungpo-gu**
**Seoul 07336 (KR)**

(72) Inventors:
- **KIM, Yu Jin**
  **Daejeon 34122 (KR)**
- **JUN, Jaemoon**
  **Daejeon 34122 (KR)**
- **YOON, Kiyoul**
  **Daejeon 34122 (KR)**
- **KIM, Gicheul**
  **Daejeon 34122 (KR)**
- **CHUNG, Ui Seok**
  **Daejeon 34122 (KR)**
- **HAN, Sangwon**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **SUPER ABSORBENT POLYMER COMPOSITION AND PREPARATION METHOD THEREOF**

(57) Provided is a superabsorbent polymer composition and a preparation method thereof. More specifically, provided is a method of preparing a superabsorbent polymer composition, the method capable of suppressing aggregation of a water-containing gel polymer and improving pulverization processability in a pulverization process by including an additive having a specific structure.

【FIG. 2】

EP 4 393 991 A1

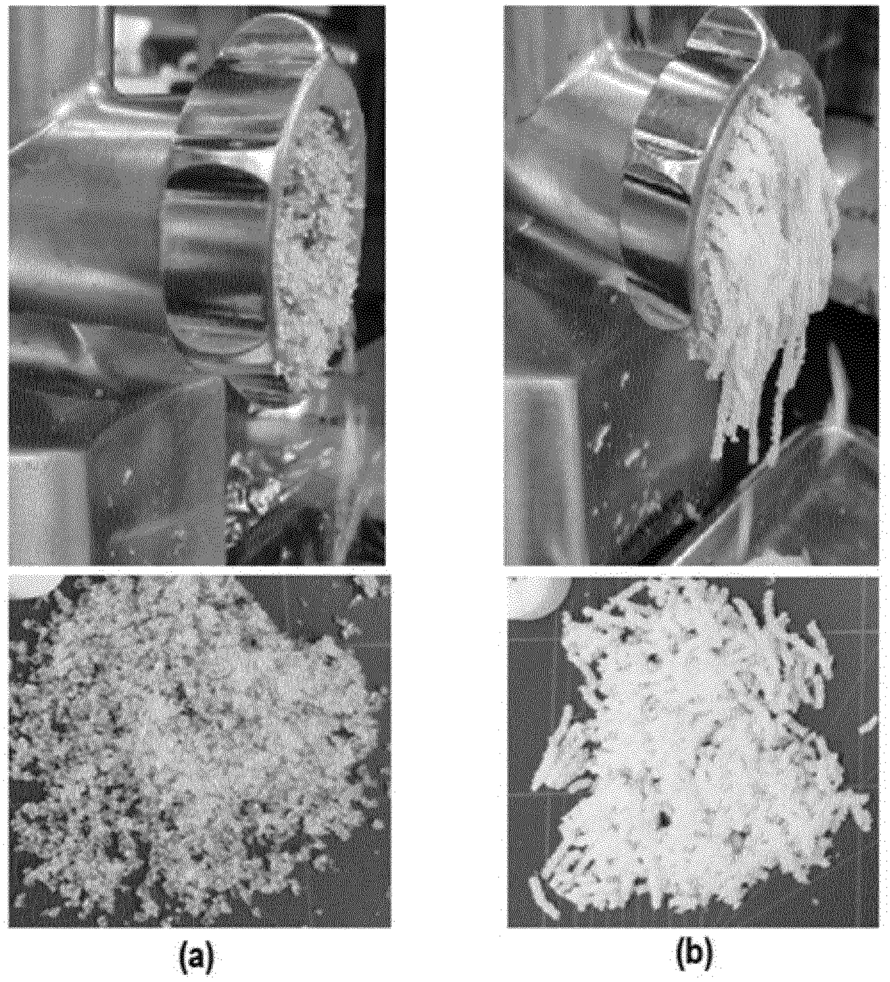

(a) (b)

## Description

[Technical Field]

Cross-reference to Related Application

The present application is based on, and claims priority from, Korean Patent Application Nos. 10-2021-0147025, and 10-2022-0140642, filed on October 29, 2021, and October 27, 2022, respectively, the disclosures of which are hereby incorporated by reference herein in their entirety.

The present invention relates to a superabsorbent polymer composition and a preparation method thereof. More specifically, the present invention relates to a method of preparing a superabsorbent polymer composition, the method capable of suppressing aggregation of a water-containing gel polymer and improving pulverization processability in a pulverization process by including an additive having a specific structure.

[Background Art]

A superabsorbent polymer (SAP) is a synthetic polymeric material capable of absorbing moisture from 500 to 1000 times its own weight. Various manufacturers have denominated it as different names, such as SAM (Super Absorbency Material), AGM (Absorbent Gel Material), etc. Since such superabsorbent polymers started to be practically applied in hygiene products, now they have been widely used for water retaining soil products for gardening, water stop materials for the civil engineering and construction, sheets for raising seedling, fresh-keeping agents for food distribution fields, materials for poultice or the like.

These superabsorbent polymers are widely used in sanitary materials such as diapers, sanitary pads, etc. Inside the sanitary materials, the superabsorbent polymer is generally distributed throughout pulp. However, recent efforts have been continuously made to provide sanitary materials such as diapers having a thinner thickness, etc., and as part of that, diapers having a reduced content of pulp, and furthermore, diapers having no pulp, so-called pulpless diapers are actively under development.

Such a sanitary material having a reduced content of pulp or having no pulp includes the superabsorbent polymer at a relatively high ratio, and the superabsorbent polymer particles are inevitably included as multiple layers in the sanitary materials. In order to allow the whole superabsorbent polymer particles included as multiple layers to more efficiently absorb a large amount of liquid such as urine, etc., it is necessary that the superabsorbent polymer basically exhibits a high absorption performance and a fast absorption rate.

On the other hand, such a superabsorbent polymer is generally prepared through the step of preparing a water-containing gel polymer containing a large amount of water by polymerizing monomers, and the step of coarsely pulverizing and drying the water-containing gel polymer, and then pulverizing the water-containing gel polymer into polymer particles having a desired particle size. In particular, during the process of coarsely pulverizing the water-containing gel polymer, there has been a problem that the coarsely pulverized water-containing gel polymers agglomerate or aggregate with each other and thus pulverization thereof does not easily occur.

Accordingly, there is a continuous demand for the development of technologies capable of improving the pulverization processability among the technologies and processes capable of improving an absorption rate of the superabsorbent polymer, in addition to improving a water retention capacity (CRC), which is a physical property representing basic absorbency and water retention capacity of the superabsorbent polymer, and an absorbency under pressure (AUP), which represents a property of well retaining absorbed liquid even under an external pressure.

[Disclosure]

[Technical Problem]

Accordingly, there is provided a method of preparing a superabsorbent polymer composition, the method capable of suppressing aggregation of a water-containing gel polymer and improving pulverization processability in a pulverization process by including an additive having a specific structure.

[Technical Solution]

To achieve the above object, there is provided a superabsorbent polymer composition, the composition including:

superabsorbent polymer particles including a crosslinked polymer of a water-soluble ethylenically unsaturated monomer having acidic groups and an internal crosslinking agent; and
an additive represented by the following Chemical Formula 1:

[Chemical Formula 1]

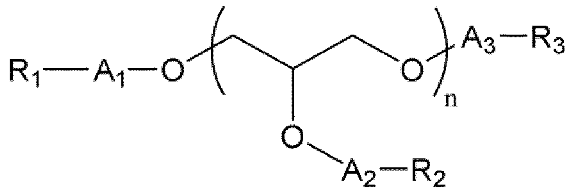

in Chemical Formula 1,
$A_1$, $A_2$, and $A_3$ are each independently a single bond, carbonyl,

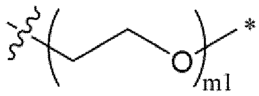,

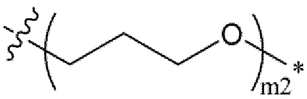 or 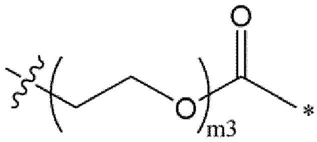,

provided that one or more thereof are carbonyl or

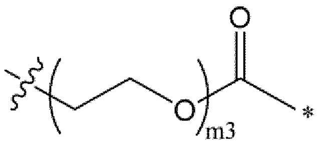,

wherein m1, m2, and m3 are each independently an integer of 1 to 8,

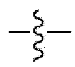

is connected to an adjacent oxygen atom,



is connected to adjacent $R_1$, $R_2$, and $R_3$, respectively,
$R_1$, $R_2$ and $R_3$ are each independently hydrogen, a straight or branched alkyl having 6 to 18 carbon atoms, or a straight or branched alkenyl having 6 to 18 carbon atoms, and
n is an integer of 1 to 9.

Further, there is provided a method of preparing a superabsorbent polymer composition, the method including the steps of:

forming a polymer by crosslinking-polymerizing a water-soluble ethylenically unsaturated monomer having acidic groups, in the presence of an internal crosslinking agent and a polymerization initiator (step 1);
neutralizing at least part of the acidic groups of the polymer (step 2);
preparing base polymer particles by micronizing the polymer in the presence of an additive represented by the following Chemical Formula 1 (step 3); and
drying the base polymer particles (step 4):

[Chemical Formula 1]

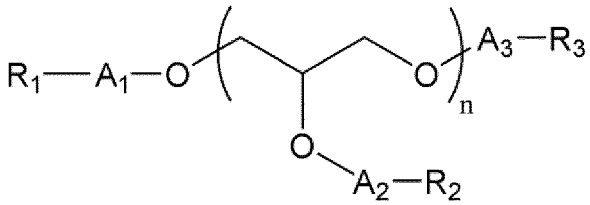

in Chemical Formula 1,
$A_1$, $A_2$, and $A_3$ are each independently a single bond, carbonyl,

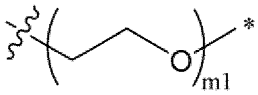
,

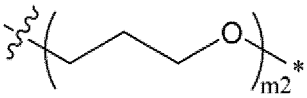
or
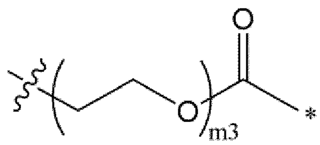
,

provided that one or more thereof are carbonyl or

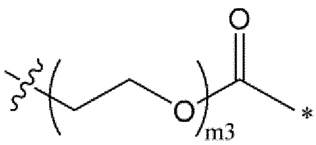
,

wherein m1, m2, and m3 are each independently an integer of 1 to 8,

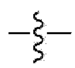

is connected to an adjacent oxygen atom,



is connected to adjacent $R_1$, $R_2$, and $R_3$, respectively,
$R_1$, $R_2$ and $R_3$ are each independently hydrogen, a straight or branched alkyl having 6 to 18 carbon atoms, or a straight or branched alkenyl having 6 to 18 carbon atoms, and
n is an integer of 1 to 9.

[Effect of the Invention]

According to a superabsorbent polymer composition of the present invention and a preparation method thereof, an additive having a specific structure is injected during a process of pulverizing a water-containing gel polymer to suppress aggregation of water-containing gel polymer particles, thereby improving pulverization processability, and as a result, absorption performances and an absorption rate of the superabsorbent polymer may be improved.

[Brief Description of Drawings]

FIG. 1 shows a flow chart showing a traditional method of preparing a superabsorbent polymer; and
FIG. 2 shows photographs showing examples of evaluation criteria in the evaluation of particle aggregation properties.

[Best Mode for Carrying Out the Invention]

The terms used in this description are just for explaining exemplary embodiments and it is not intended to restrict the present invention. The singular expression may include the plural expression unless it is differently expressed contextually. It must be understood that the term "include", "equip", or "have" in the present description is only used for designating the existence of characteristics taken effect, steps, components, or combinations thereof, and do not exclude the existence or the possibility of addition of one or more different characteristics, steps, components, or combinations thereof beforehand.
The present invention may be variously modified and have various forms, and specific exemplary embodiments are exemplified and explained in detail in the following description. However, it is not intended to limit the present invention to the specific exemplary embodiments, and it must be understood that the present invention includes every modifications, equivalents, or replacements included in the spirit and technical scope of the present invention.
Hereinafter, a method of preparing a superabsorbent polymer and the superabsorbent polymer will be described in more detail according to specific embodiments of the present invention.
Prior to that, the technical terms used herein are only for mentioning particular embodiments and is not intended to be limiting of the present invention. Further, the singular expression used herein may include the plural expression unless it is differently expressed contextually.
As used herein, the term "polymer" refers to those in a polymerized state of a water-soluble ethylenically unsaturated monomer, and may include all water content ranges or particle size ranges. Among the above polymers, a polymer having a water content (moisture content) of about 40% by weight or more after polymerizing before drying may be referred to as a water-containing gel polymer, and particles obtained by pulverizing and drying such a water-containing gel polymer may be referred to as a crosslinked polymer.
Further, the term "superabsorbent polymer particle" refers to a material in the form of particles, the material including the crosslinked polymer which is obtained by polymerizing the water-soluble ethylenically unsaturated monomer including acidic groups, of which at least part is neutralized, and crosslinking the same by an internal crosslinking agent.
Further, the term "superabsorbent polymer" refers to, depending on the context, the crosslinked polymer obtained by polymerizing the water-soluble ethylenically unsaturated monomer including acidic groups of which at least part is neutralized, or a base polymer in the form of powder consisting of superabsorbent polymer particles obtained by pulverizing the crosslinked polymer, or is used to encompass those made suitable for commercialization by performing an additional process on the crosslinked polymer or the base polymer, for example, surface crosslinking, re-assembling of fine particles, drying, pulverizing, classifying, etc. Therefore, the term "superabsorbent polymer composition" may be interpreted as a composition including the superabsorbent polymer, i.e., a plurality of superabsorbent polymer particles.
Further, the term "fine particle" refers to a particle having a particle size of less than 150 $\mu$m among superabsorbent polymer particles. The particle size of the polymer particle may be measured according to the European Disposables and Nonwovens Association (EDANA) standard WSP 220.3 method.
Further, the term "chopping" refers to fragmenting the water-containing gel polymer into small pieces of millimeter sizes in order to increase the drying efficiency, and is distinguished from pulverizing to a particle size of the micrometer or normal particle level.
In addition, the term "micronizing (micronization)" refers to pulverizing the water-containing gel polymer into a particle size of tens to hundreds of micrometers, and is distinguished from "chopping".
Meanwhile, the superabsorbent polymer is traditionally prepared by forming the water-containing gel polymer by crosslinking polymerization of the water-soluble ethylenically unsaturated monomer having at least partially neutralized acidic groups in the presence of an internal crosslinking agent and a polymerization initiator, drying the water-containing gel polymer thus formed, and then pulverizing the water-containing gel polymer into a desired particle size. At this time,

in order to facilitate drying of the water-containing gel polymer and to increase efficiency of the pulverizing process, the chopping process is commonly performed, in which the water-containing gel polymer is chopped into particles of several millimeter sizes, before the drying process. However, in this chopping process, due to stickiness of the water-containing gel polymer, the water-containing gel polymer is not pulverized into the micro-sized particles and it becomes an aggregated gel. When this aggregated gel-type water-containing gel polymer is dried, a sheet-shaped dry product is formed, and in order to pulverize the sheet-shaped dry product into micro-sized particles, a multi-stage pulverization process should be performed. In this process, there has been a problem that many fine particles are generated.

Specifically, a flow chart showing a traditional method of preparing a superabsorbent polymer is illustrated in FIG. 1. Referring to FIG. 1, the superabsorbent polymer has been traditionally prepared by the following steps:

(Neutralizing) step of neutralizing at least part of acidic groups of a water-soluble ethylenically unsaturated monomer;
(Polymerizing) step of forming a water-containing gel polymer by crosslinking-polymerizing the water-soluble ethylenically unsaturated monomer having at least partially neutralized acidic groups, in the presence of an internal crosslinking agent and a polymerization initiator;
(Chopping) step of chopping the water-containing gel polymer;
(Drying) step of drying the chopped water-containing gel polymer; and
(Pulverizing/Classifying) step of pulverizing the dried polymer and then classifying the same into normal particles and fine particles.

As described above, the chopped water-containing gel polymer has an aggregated gel shape having a size of about 1 cm to about 10 cm, and the chopped water-containing gel polymer is stacked on a belt of which bottom consists of perforated plates, and dried by hot air supplied from the lower or upper part. Since the polymer dried by the drying method exhibits a sheet shape rather than a particle shape, the step of classifying after pulverizing has been performed by the step of coarsely pulverizing the prepared particles into normal particles, i.e., particles having a particle size of 150 $\mu$m to 850 $\mu$m, and then classifying the same, followed by fine pulverizing and classifying. Since the amount of fine particles separated in the final classifying step by such a preparation method is as large as about 10% by weight to about 20% by weight with respect to the total weight of the superabsorbent polymer finally prepared, the separated fine particles are reused in such a way that they are mixed with an appropriate amount of water to reassemble the fine particles, which are then injected in the chopping step or the step before drying.

However, when the fine particle reassembly mixed with water for the reuse of the fine particles is re-injected into the pulverizing or drying process, there has been a problem of causing an increase in equipment load and/or energy consumption, and the fine particles which remain unclassified have caused deterioration of physical properties of the superabsorbent polymer.

Accordingly, the present inventors have identified that the amount of fine particles generated in the traditional preparation method heavily influences the pulverization process, and they found that when the fine particles are prepared in an aggregated form of particles by adding a surfactant in the process of chopping the fine particles (micronization process) to pulverize the fine particles more finely than before, and at the same time, to control aggregation, the amount of fine particles generated during the preparation process may be remarkably reduced.

Meanwhile, when the surfactant is added to the neutralized water-containing gel polymer, the surfactant penetrates into the water-containing gel polymer rather than existing at the interface of the water-containing gel polymer, due to the high water content of the neutralized water-containing gel polymer, and thus the surfactant does not properly perform its role.

Many studies have been conducted to solve this problem, and as a result, it was found that, unlike in the common method of preparing the superabsorbent polymer, in which polymerization is carried out in a state in which the acidic groups of the water-soluble ethylenically unsaturated monomer are neutralized, when the polymer is obtained by performing polymerization in a state in which the acidic groups are not neutralized, the water-containing gel polymer is micronized in the presence of the surfactant, and then the acidic groups of the polymer are neutralized, or when the water-containing gel polymer is formed by neutralizing the acidic groups of the polymer, and then the water-containing gel polymer is micronized in the presence of the surfactant, or when micronizing is performed, and at the same time, the acidic groups present in the polymer are neutralized, a large amount of the surfactant is present on the surface of the polymer to lower the high stickiness of the polymer, thereby sufficiently playing its role of preventing excessive aggregation of the polymer and controlling the aggregation state at a desired level.

Accordingly, the polymer is prepared as secondary particles in which the primary particles are aggregated, and then the pulverizing and drying processes are performed under milder conditions, and therefore, the amount of fine particles generated during the processes may be remarkably reduced.

In addition, when the polymer is pulverized in the presence of the additive having the specific structure of Chemical Formula 1 as described above, the hydrophobic functional moiety included in the additive imparts hydrophobicity to the surface of the pulverized superabsorbent polymer particles to relieve the frictional force between particles. Accordingly, while increasing the bulk density of the superabsorbent polymer, the hydrophilic functional moiety included in the additive is also bound to the superabsorbent polymer particles so that the surface tension of the polymer does not decrease. Accordingly, the superabsorbent polymer prepared according to the above-described preparation method may have a high bulk density value while exhibiting a surface tension equivalent to those of polymers prepared without using such an additive.

In addition, when the polymer is formed by performing polymerization in an unneutralized state, and then the acidic groups present in the polymer are neutralized, it is possible to form a longer chain polymer and to achieve the effect of reducing the content of water-soluble components that exist in an uncrosslinked state due to incomplete crosslinking.

The water-soluble components have a property of being easily eluted when the superabsorbent polymer comes into contact with a liquid, and therefore, when the content of the water-soluble components is high, most of the eluted water-soluble components remain on the surface of the superabsorbent polymer and makes the superabsorbent polymer sticky, causing a reduction in the liquid permeability. Therefore, in terms of liquid permeability, it is important to maintain the low content of water-soluble components.

In summary, the present inventors confirmed that when the polymer is pulverized by mixing with the additive having the specific structure represented by the following Chemical Formula 1, the aggregation of the pulverized water-containing gel polymer particles is suppressed to improve the pulverization processability, thereby completing the present invention. Particularly, the particles included in the superabsorbent polymer composition prepared according to the above preparation method are charactered by exhibiting an improved absorption rate, as compared to the case where the additive is not used.

[Chemical Formula 1]

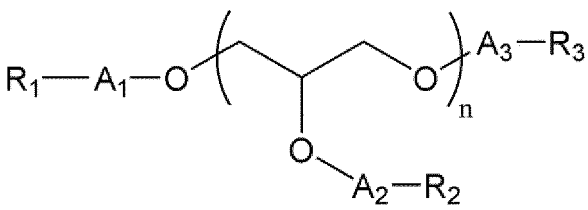

in Chemical Formula 1,

$A_1$, $A_2$, and $A_3$ are each independently a single bond, carbonyl,

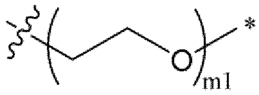

,

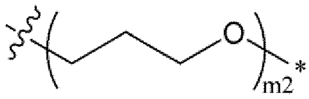

or

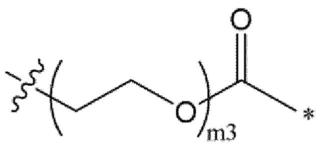

,

provided that one or more thereof are carbonyl or

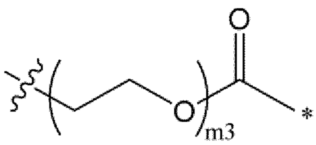

,

wherein m1, m2, and m3 are each independently an integer of 1 to 8,

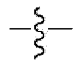

is connected to an adjacent oxygen atom,



is connected to adjacent $R_1$, $R_2$, and $R_3$, respectively,
$R_1$, $R_2$ and $R_3$ are each independently hydrogen, a straight or branched alkyl having 6 to 18 carbon atoms, or a straight or branched alkenyl having 6 to 18 carbon atoms, and
n is an integer of 1 to 9.

Specifically, the additive represented by the Chemical Formula 1 has a hydrophobic functional group and a hydrophilic functional group at the same time. On the other hand, since the water-soluble ethylenically unsaturated monomer contains acidic groups (-COOH) and/or neutralized acidic groups (-COO-), acidic groups (-COOH) that remain without participating in the polymerization and/or the hydrophilic moiety by the neutralized acidic groups (-COO-) exist in a large amount on the surface of the polymer prepared by polymerization. Therefore, when the additive is mixed with the polymer, the hydrophilic functional group of the additive is adsorbed onto at least part of the hydrophilic moiety present on the surface of the polymer, and the surface of the polymer onto which the additive is adsorbed exhibits a hydrophobic property by the hydrophobic functional group located at the other end of the additive. Accordingly, aggregation between the pulverized water-containing gel polymer particles may be suppressed.

Hereinafter, respective components of the superabsorbent polymer composition of one embodiment will be explained in more detail.

**(Superabsorbent polymer composition)**

According to one embodiment of the present invention, provided is a superabsorbent polymer composition, the composition including:

superabsorbent polymer particles including a crosslinked polymer of a water-soluble ethylenically unsaturated monomer having acidic groups and an internal crosslinking agent; and an additive represented by the following Chemical Formula 1:

[Chemical Formula 1]

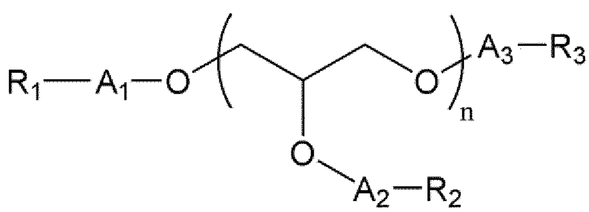

in Chemical Formula 1,
$A_1$, $A_2$, and $A_3$ are each independently a single bond, carbonyl,

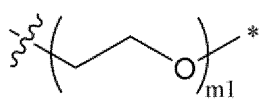

,

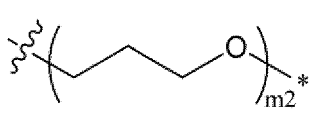

or

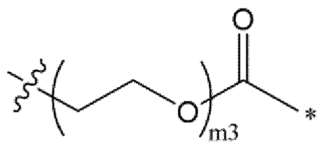

,

provided that one or more thereof are carbonyl or

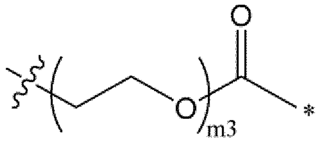

,

wherein m1, m2, and m3 are each independently an integer of 1 to 8,

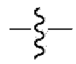

is connected to an adjacent oxygen atom,



is connected to adjacent $R_1$, $R_2$, and $R_3$, respectively,

$R_1$, $R_2$ and $R_3$ are each independently hydrogen, a straight or branched alkyl having 6 to 18 carbon atoms, or a straight or branched alkenyl having 6 to 18 carbon atoms, and

n is an integer of 1 to 9.

The superabsorbent polymer composition of one embodiment includes superabsorbent polymer particles including the crosslinked polymer of the water-soluble ethylenically unsaturated monomer having acidic groups and the internal crosslinking agent. In this regard, the crosslinked polymer is obtained by crosslinking polymerization of the water-soluble ethylenically unsaturated monomer having acidic groups in the presence of the internal crosslinking agent, and has a three-dimensional network structure in which main chains formed by polymerization of the monomers are crosslinked by the internal crosslinking agent.

In other words, the superabsorbent polymer composition of one embodiment includes superabsorbent polymer particles including the crosslinked polymer of the water-soluble ethylenically unsaturated monomer having acidic groups and the internal crosslinking agent. As described, when the crosslinked polymer has the three-dimensional network structure in which main chains formed by polymerization of the monomers are crosslinked by the internal crosslinking agent, water retention capacity and absorbency under pressure, which are overall physical properties of the superabsorbent polymer, may be remarkably improved, as compared to those having a two-dimensional linear structure in which the additional crosslinking by the internal crosslinking agent is not performed.

The water-soluble ethylenically unsaturated monomer may be any monomer commonly used in the preparation of superabsorbent polymers. For non-limiting example, the water-soluble ethylenically unsaturated monomer may be a compound represented by the following Chemical Formula 2:

[Chemical Formula 2] R-COOM'

in Chemical Formula 2,

R is a C2-5 alkyl group containing an unsaturated bond, and

M' is a hydrogen atom, a monovalent or divalent metal, an ammonium group, or an organic amine salt.

Preferably, the monomer may be one or more selected from the group consisting of (meth)acrylic acid, and a monovalent (alkali) metal salt, a divalent metal salt, an ammonium salt, and an organic amine salt thereof.

When (meth)acrylic acid and/or a salt thereof is used as the water-soluble ethylenically unsaturated monomer, it is advantageous in that a superabsorbent polymer having improved absorption property may be obtained. In addition, as the monomer, maleic anhydride, fumaric acid, crotonic acid, itaconic acid, 2-acryloylethanesulfonic acid, 2-methacryloylethanesulfonic acid, 2-(meth)acryloylpropane sulfonic acid, or 2-(meth)acrylamido-2-methylpropane sulfonic acid, (meth)acrylamide, N-substituted (meth)acrylate, 2-hydroxyethyl(meth)acrylate, 2-hydroxypropyl(meth)acrylate, methoxypolyethylene glycol(meth)acrylate, polyethylene glycol (meth)acrylate, (N,N)-dimethylaminoethyl(meth)acrylate, (N,N)-dimethylaminopropyl(meth)acrylamide, etc. may be used.

Here, the water-soluble ethylenically unsaturated monomer has acidic groups, of which at least part is neutralized by a neutralizing agent in the neutralization step to be described later. In this regard, as the neutralizing agent, basic substances capable of neutralizing acidic groups, such as sodium hydroxide, potassium hydroxide, ammonium hydroxide, etc., may be used, which will be described in more detail in the preparation method to be described later.

Further, as used herein, the term 'internal crosslinking agent' is a term used to distinguish it from a surface crosslinking agent for crosslinking the surface of the superabsorbent polymer particles, described later, and the internal crosslinking agent functions to polymerize the water-soluble ethylenically unsaturated monomers by crosslinking the unsaturated bonds thereof. The crosslinking in the above step occurs regardless of the surface or inside of the polymer. However, through the surface crosslinking process of the superabsorbent polymer particles described later, the particle surface of the superabsorbent polymer finally prepared has a structure crosslinked by the surface crosslinking agent, and the inside thereof has a structure crosslinked by the internal crosslinking agent.

As the internal crosslinking agent, any compound may be used as long as it enables introduction of crosslinking bonds during polymerization of the water-soluble ethylenically unsaturated monomer. For non-limiting example, as the internal crosslinking agent, multifunctional crosslinking agents, such as N,N'-methylenebisacrylamide, trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentacrylate, glycerin tri(meth)acrylate, pentaerythritol tetraacrylate, triarylamine, pentaerythritol triallyl ether, ethylene glycol diglycidyl ether, propylene glycol, glycerin, or ethylene carbonate may be used alone or in combination of two or more thereof, but are not limited thereto. Among them, pentaerythritol triallyl ether may be preferably used.

The crosslinking polymerization of the water-soluble ethylenically unsaturated monomer in the presence of the internal crosslinking agent may be performed by thermal polymerization, photo-polymerization, or hybrid polymerization in the presence of a polymerization initiator, if necessary, a thickener, a plasticizer, a preservation stabilizer, an antioxidant, etc.

Such superabsorbent polymer particles may have a particle size of about 150 $\mu$m to about 850 $\mu$m, and the particle size may be measured according to European Disposables and Nonwovens Association (EDANA) standard WSP 220.3 method.

Further, the superabsorbent polymer composition includes the additive represented by Chemical Formula 1. As described above, the additive is mixed with the polymer, which is added such that the micronization (chopping) step may be easily accomplished without aggregation.

The additive represented by Chemical Formula 1 is a nonionic surfactant and has excellent surface adsorption performance by hydrogen bonding force even with an unneutralized polymer, and therefore, it is suitable for realizing the desired aggregation control effect. In contrast, when not a nonionic surfactant but an anionic surfactant is mixed with a polymer neutralized with a neutralizing agent such as NaOH or $Na_2SO_4$, it is adsorbed onto carboxyl substituents of the polymer via ionized $Na^+$ ions, and when mixed with the unneutralized polymer, there is a problem in that the adsorption efficiency for the polymer is relatively lowered due to competition with the anions of the carboxyl substituents of the polymer.

Specifically, in the additive represented by Chemical Formula 1, the hydrophobic functional group is the terminal functional groups, $R_1$, $R_2$, $R_3$ moieties (if not hydrogen), and the hydrophilic functional group further includes a glycerol-derived moiety in the chain and a hydroxyl group at the terminal (when $A_n$ is a single bond, and at the same time, when $R_n$ is hydrogen, n=1 to 3), wherein the glycerol-derived moiety and the terminal hydroxyl group are hydrophilic functional groups and serve to improve adsorption performance on the polymer surface. Accordingly, aggregation of the superabsorbent polymer particles may be effectively suppressed.

In Chemical Formula 1, the hydrophobic functional groups, $R_1$, $R_2$, $R_3$ moieties (if not hydrogen) are each independently a straight or branched alkyl having 6 to 18 carbon atoms, or a straight or branched alkenyl having 6 to 18 carbon atoms. In this regard, when $R_1$, $R_2$, and $R_3$ moieties (if not hydrogen) are alkyl or alkenyl having less than 6 carbon atoms, there is a problem in that aggregation of the pulverized particles may not be effectively controlled because the chain length is short. When $R_1$, $R_2$, and $R_3$ moieties (if not hydrogen) are alkyl or alkenyl having more than 18 carbon atoms, there may be problems in that the additive may not be effectively mixed with the polymer because its mobility is reduced, and the unit price of the composition is increased due to the increased cost of the additive.

Preferably, when $R_1$, $R_2$, and $R_3$ may be hydrogen or a straight or branched alkyl having 6 to 18 carbon atoms, they may be 2-methylhexyl, n-heptyl, 2-methylheptyl, n-octyl, n-nonyl, n-decanyl, n-undecanyl, n-dodecanyl, n-tridecanyl, n-tetradecanyl, n-pentadecanyl, n-hexadecanyl, n-heptadecanyl, or n-octadecanyl, or when $R_1$, $R_2$, and $R_3$ may be a straight or branched alkenyl having 6 to 18 carbon atoms, they may be 2-hexenyl, 2-heptenyl, 2-octenyl, 2-nonenyl, n-decenyl, 2-undecenyl, 2-dodecenyl, 2-tridecenyl, 2-tetradecenyl, 2-pentadecenyl, 2-hexadecenyl, 2-heptadecenyl, or 2-octadecenyl.

The additive may be selected from compounds represented by the following Chemical Formula 1-1 to Chemical Formula 1-14:

[Chemical Formula 1-1]

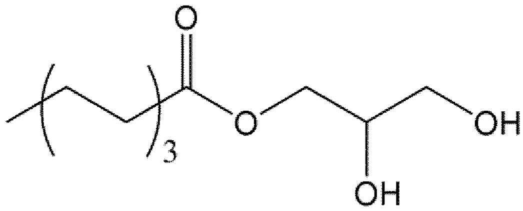

[Chemical Formula 1-2]

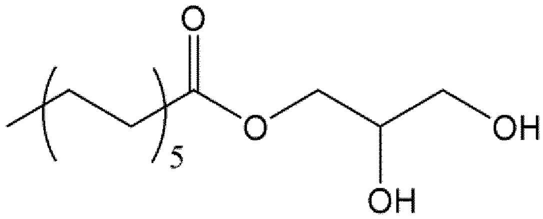

[Chemical Formula 1-3]

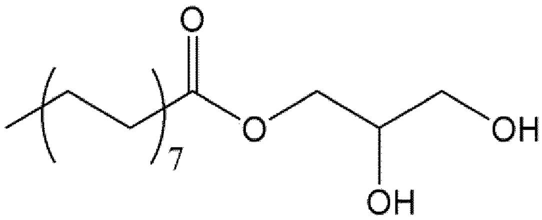

[Chemical Formula 1-4]

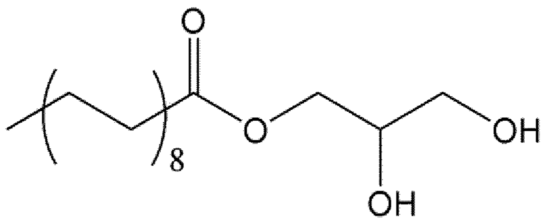

[Chemical Formula 1-5]

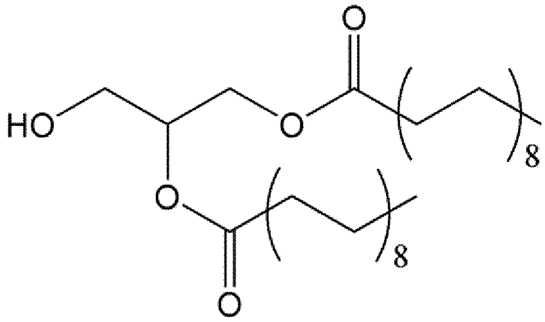

[Chemical Formula 1-6]

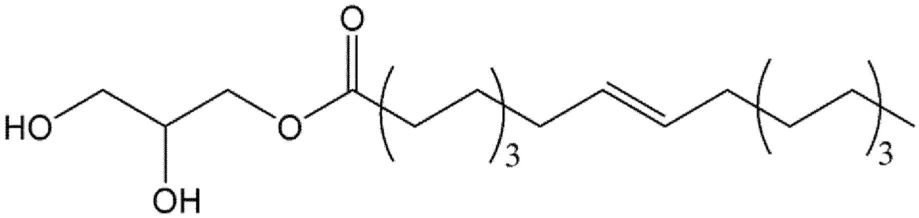

[Chemical Formula 1-7]

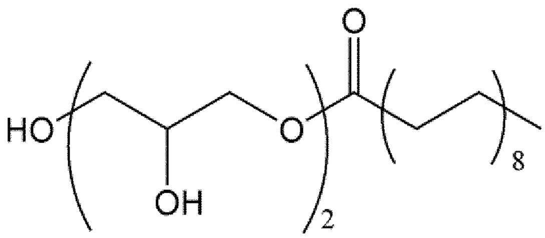

[Chemical Formula 1-8]

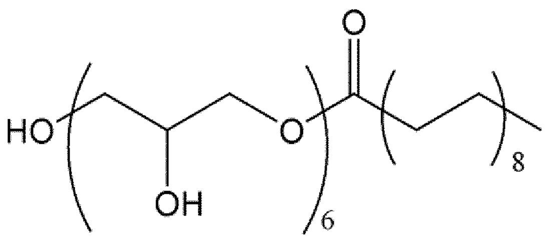

[Chemical Formula 1-9]

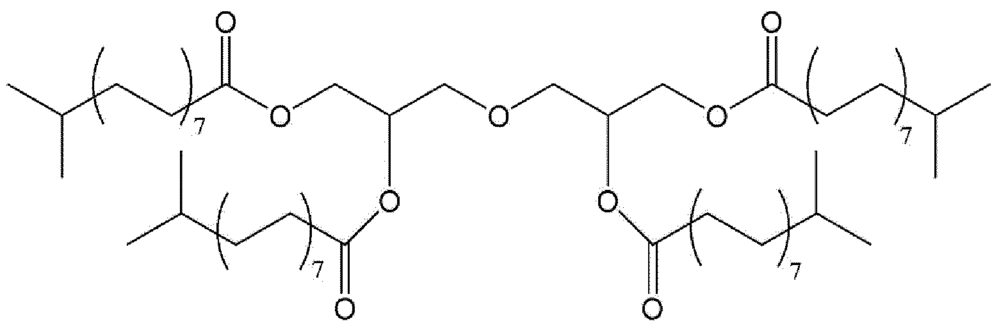

[Chemical Formula 1-10]

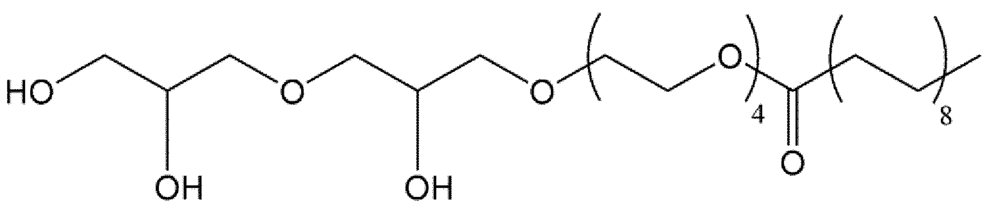

[Chemical Formula 1-11]

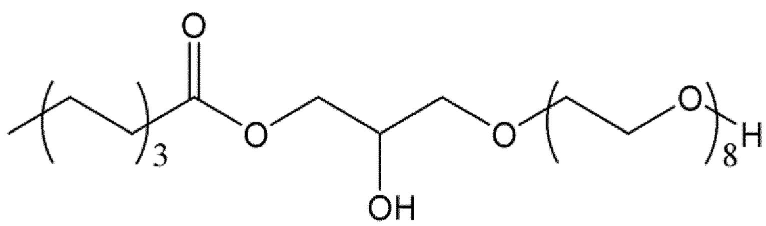

[Chemical Formula 1-12]

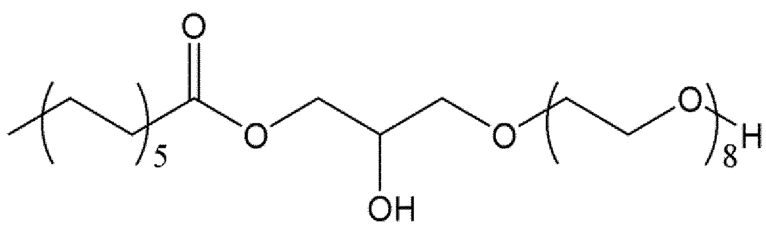

[Chemical Formula 1-13]

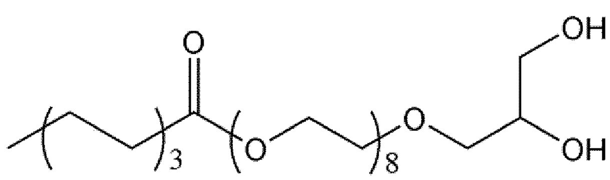

[Chemical Formula 1-14]

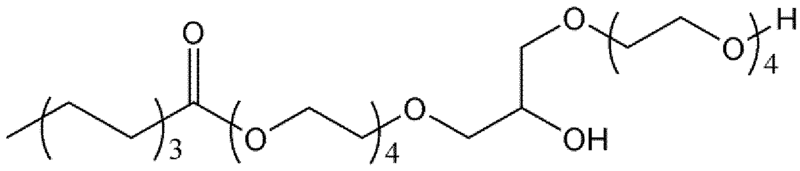

The additive may be included in an amount of 0.01 part by weight to 10 parts by weight with respect to 100 parts by weight of the water-soluble ethylenically unsaturated monomer. When the total content of the additive, relative to the monomer, in the composition is excessively low, the effect of controlling aggregation by the additive is small, and thus superabsorbent polymer particles which are not pulverized into a desired particle size may be included. When the total content of the additive is excessively high, a balance of water retention capacity and absorbency under pressure which are the overall physical properties of the superabsorbent polymer may deteriorate.

The content of the additive in the superabsorbent polymer composition may be measured by adding 1 g of the superabsorbent polymer composition to 1 ml of distilled water, sufficiently mixing for 1 hour until swelling, extracting only the solution part by filtration, and then analyzing the content of the additive dissolved in the solution part by performing HPLC analysis.

More specifically, the additive may be included in an amount of 0.01 part by weight or more, 0.02 parts by weight or more, 0.05 parts by weight or more, 0.1 parts by weight or more, or 0.5 parts by weight or more, and 10 parts by weight or less, 8 parts by weight or less, 5 parts by weight or less, 3 parts by weight or less, or 2 parts by weight or less with respect to 100 parts by weight of the water-soluble ethylenically unsaturated monomer.

Meanwhile, at least part of the additive may be present on the surface of the superabsorbent polymer particles. Here, "at least part of the additive is present on the surface of the superabsorbent polymer particles" means that at least part of the additive is adsorbed or bound onto the surface of the superabsorbent polymer particles. Specifically, the additive may be physically or chemically adsorbed onto the surface of the superabsorbent polymer. More specifically, the hydrophilic functional group of the additive may be physically adsorbed onto the hydrophilic moiety of the surface of the superabsorbent polymer by intermolecular forces such as dipole-dipole interaction. As described, the hydrophilic moiety of the additive is physically adsorbed onto the surface of the superabsorbent polymer particles to cover the surface, and the hydrophobic moiety of the additive is not adsorbed onto the surface of the polymer particles, and thus the surface of the polymer particles may be coated with the additive in a kind of micelle structure.

Therefore, when at least part of the additive is present on the surface of the superabsorbent polymer particles, the aggregation phenomenon, in which the pulverized particles aggregate with each other during the process of preparing the superabsorbent polymer composition, may be more effectively suppressed, as compared to the case where the whole additive of Chemical Formula 1 are present inside the superabsorbent polymer particles, specifically, inside the crosslinked polymer.

Further, as at least part of the additive is present on the surface of the superabsorbent polymer particles, the superabsorbent polymer composition including the additive may have an improved absorption rate, as compared to the composition without the additive.

Meanwhile, when the superabsorbent polymer composition does not further include a surface-crosslinked layer described later, it may not include other hydrophilic additive than a plurality of the superabsorbent polymer particles, the additive, and additive hydrolysates which are generated by hydrolysis of the additive during the process of preparing the superabsorbent polymer.

Specifically, the superabsorbent polymer composition of one embodiment may not include a compound having a plurality of hydroxyl group-containing glucose units in a molecule, such as microcrystalline cellulose. For example, when the superabsorbent polymer composition includes microcrystalline cellulose having an average particle size of 1 μm to 10 μm, such as AVICEL® PH-101 represented by the following Chemical Formula 3 available from FMC, aggregation between the superabsorbent polymer particles may not be suppressed due to a plurality of the hydroxyl groups, and thus the above-described effects of the additive may not be effectively expressed.

[Chemical Formula 3]

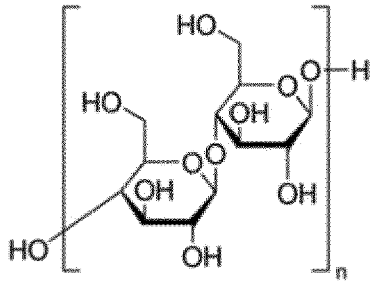

Further, the superabsorbent polymer composition of one embodiment may not include hydrophilic additives, such as polyethylene glycol, polypropylene glycol, poly(ethylene glycol)-poly(propylene glycol) copolymer, polyoxyethylene lauryl ether carboxylic acid, sodium polyoxyethylene lauryl ether carboxylate, lauryl sulfate, sodium lauryl sulfate, etc. Since such additives are not sufficiently adsorbed onto the surface of the crosslinked polymer, there is a problem in that aggregation between superabsorbent polymer particles is not effectively suppressed. Accordingly, when the superabsorbent polymer composition includes the hydrophilic additive as described above instead of the additive of Chemical Formula 1, aggregation between particles is not suppressed after pulverization of the crosslinked polymer, and thus the superabsorbent polymer composition includes a large amount of fine particles and exhibits low water retention capacity and low bulk density.

Meanwhile, the superabsorbent polymer composition may further include a surface-crosslinked layer which is formed on at least part of the surface of the superabsorbent polymer particles by further crosslinking the crosslinked polymer via a surface crosslinking agent. This is to increase the surface crosslinking density of the superabsorbent polymer particles. When the superabsorbent polymer particles further include the surface-crosslinked layer as described above, they may have a structure having a higher crosslinking density on the outside than the inside.

As the surface crosslinking agent, any surface crosslinking agent that has been traditionally used in the preparation of superabsorbent polymers may be used without any particular limitation. For example, the surface crosslinking agent may include one or more polyols selected from the group consisting of ethylene glycol, propylene glycol, 1,3-propanediol, 1,4-butanediol, 1,6-hexanediol, 1,2-hexanediol, 1,3-hexanediol, 2-methyl-1,3-propanediol, 2,5-hexanediol, 2-methyl-1,3-pentanediol, 2-methyl-2,4-pentanediol, tripropylene glycol and glycerol; one or more carbonate-based compounds selected from the group consisting of ethylene carbonate, propylene carbonate, and glycerol carbonate; an epoxy compound such as ethylene glycol diglycidyl ether; an oxazoline compound such as oxazolidinone, etc.; a polyamine compound; an oxazoline compound; a mono-, di- or poly-oxazolidinone compound; a cyclic urea compound; and the like.

Specifically, as the surface crosslinking agent, one or more, two or more, or three or more of the aforementioned surface crosslinking agents may be used. For example, propylene glycol, ethylene carbonate, propylene carbonate may be used.

Further, the superabsorbent polymer composition may have an absorption rate (vortex time) of 30 seconds or less, 27 seconds or less, 25 seconds or less, 20 seconds or less, 15 seconds or less, or 12 seconds or less, as measured at 24.0°C according to the vortex method. Further, as the value of the absorption rate is smaller, it is superior, and thus the lower limit of the absorption rate is 0 second in theory. For example, it may be about 5 seconds or more, or about 8 seconds or more, or about 10 seconds or more. In this regard, the method of measuring the absorption rate of the superabsorbent polymer will be explained in more detail in Experimental Example described later.

Further, the superabsorbent polymer composition may have a water retention capacity (CRC) in the range of 39 g/g or more or 40 g/g or more, or 50 g/g or less, or 48 g/g or less, or 45 g/g or less, as measured according to the EDANA method WSP 241.3. The method of measuring the water retention capacity will be explained in more detail in Experimental Example described later.

Further, the superabsorbent polymer composition may have absorbency under pressure (AUP) of 0.7 psi of 24.0 g/g or more, 25.0 g/g or more, 26.0 g/g or more, or 24.3 g/g or more, and 30 g/g or less, 28.0 g/g or less, or 26.0 g/g or less, as measured according to the EDANA method WSP 242.3. The method of measuring the absorbency under pressure will be explained in more detail in Experimental Example described later.

**(Method of preparing superabsorbent polymer composition)**

Meanwhile, the superabsorbent polymer composition may be prepared by including the steps of:

forming a polymer by crosslinking-polymerizing a water-soluble ethylenically unsaturated monomer having acidic groups, in the presence of an internal crosslinking agent and a polymerization initiator (step 1);
neutralizing at least part of the acidic groups of the polymer (step 2);
preparing base polymer particles by micronizing the polymer in the presence of an additive represented by the following Chemical Formula 1 (step 3); and
drying the base polymer particles (step 4):

[Chemical Formula 1]

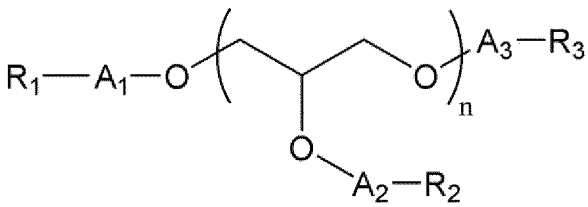

in Chemical Formula 1,
$A_1$, $A_2$, and $A_3$ are each independently a single bond, carbonyl,

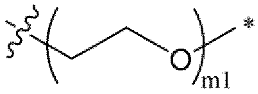

,

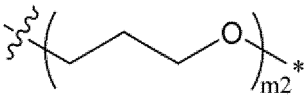

or

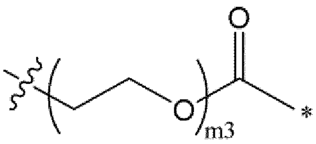

,

provided that one or more thereof are carbonyl or

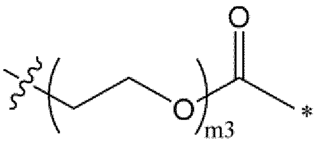

,

wherein m1, m2, and m3 are each independently an integer of 1 to 8,

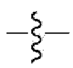

is connected to an adjacent oxygen atom,



is connected to adjacent $R_1$, $R_2$, and $R_3$, respectively,
$R_1$, $R_2$ and $R_3$ are each independently hydrogen, a straight or branched alkyl having 6 to 18 carbon atoms, or a

straight or branched alkenyl having 6 to 18 carbon atoms, and
n is an integer of 1 to 9.

Hereinafter, respective steps of the method of preparing the superabsorbent polymer of one embodiment will be explained in more detail.

**(Step 1)**

In the method of preparing the superabsorbent polymer according to one embodiment, the step of forming the water-containing gel polymer by crosslinking-polymerizing the water-soluble ethylenically unsaturated monomer having acidic groups in the presence of the internal crosslinking agent and the polymerization initiator is first performed.
The step may consist of a step of preparing a monomer composition by mixing the water-soluble ethylenically unsaturated monomer, the internal crosslinking agent and the polymerization initiator, and a step of forming the polymer by performing thermal polymerization or photopolymerization of the monomer composition. In this regard, descriptions of the water-soluble ethylenically unsaturated monomer and internal crosslinking agent refer to those described above.
Here, the water-soluble ethylenically unsaturated monomer has acidic groups. As described above, in the traditional preparation of the superabsorbent polymer, the water-containing gel polymer has been formed by crosslinking polymerization of monomers in which at least part of the acidic groups are neutralized by a neutralizing agent. Specifically, in the step of mixing the water-soluble ethylenically unsaturated monomer having the acidic group, the internal crosslinking agent, the polymerization initiator, and the neutralizing agent, at least part of the acidic groups of the water-soluble ethylenically unsaturated monomer was neutralized.
However, according to one embodiment of the present invention, the polymer is formed by first performing polymerization in a state where the acidic groups of the water-soluble ethylenically unsaturated monomer are not neutralized.
The water-soluble ethylenically unsaturated monomer (e.g., acrylic acid), of which acidic groups are not neutralized, is in a liquid state at room temperature, and since its miscibility with a solvent (water) is high, it exists as a mixed solution in the monomer composition. However, the water-soluble ethylenically unsaturated monomer, of which acidic groups are neutralized, is in a solid state at room temperature, and has different solubilities depending on the temperature of the solvent (water), and its solubility is lower as temperature is lower.
As described, the water-soluble ethylenically unsaturated monomer, of which acidic groups are not neutralized, has high solubility or miscibility for a solvent (water), as compared to the monomer of which acid groups are neutralized, and thus it does not precipitate even at a low temperature. Accordingly, it is advantageous for a long-time polymerization at a low temperature. Accordingly, a polymer having a high molecular weight and a uniform molecular weight distribution may be stably formed by performing polymerization for a long time using the water-soluble ethylenically unsaturated monomer, of which acidic groups are not neutralized.
Further, it is possible to form a longer-chain polymer, and thus it is possible to achieve the effect of reducing the content of the water-soluble components present in an uncrosslinked state due to incomplete polymerization or crosslinking.
Further, as described, when the monomer, of which acidic groups are not neutralized, is first polymerized to form the polymer, and after neutralization, micronization is performed in the presence of the additive of Chemical Formula 1, or when micronization is performed in the presence of the additive of Chemical Formula 1, followed by neutralization, or when micronization and neutralization of the acidic groups present in the polymer are performed at the same time, a large amount of the additive of Chemical Formula 1 is present on the surface of the polymer, and may sufficiently play a role in lowering the stickiness of polymer.
With regard to the kind of the water-soluble ethylenically unsaturated monomer in the monomer composition, those described in the above description of the superabsorbent polymer composition are also applied. Further, the concentration of the water-soluble ethylenically unsaturated monomer may be appropriately controlled in consideration of the polymerization time, the reaction conditions, etc., and the concentration may be about 20% by weight to about 60% by weight, or about 20% by weight to about 40% by weight.
With regard to the kind of the internal crosslinking agent in the monomer composition, those described in the above description of the superabsorbent polymer composition are also applied. Further, the content of the internal crosslinking agent may be 0.01 part by weight to 5 parts by weight with respect to 100 parts by weight of the water-soluble ethylenically unsaturated monomer. For example, the internal crosslinking agent may be used in an amount of 0.01 part by weight or more, 0.05 parts by weight or more, or 0.1 part by weight or more, and 5 parts by weight or less, 3 parts by weight or less, 2 parts by weight or less, 1 part by weight or less, or 0.7 parts by weight or less with respect to 100 parts by weight of the water-soluble ethylenically unsaturated monomer. When the content of the internal crosslinking agent is too low, crosslinking does not occur sufficiently, and thus it may be difficult to achieve the strength above the appropriate level, and when the content of the internal crosslinking agent is too high, it may be difficult to achieve

the desired water retention capacity due to the increased internal crosslinking density.

Further, the polymerization initiator is appropriately selected according to the polymerization method. When a thermal polymerization method is employed, a thermal polymerization initiator is used. When a photo-polymerization method is employed, a photo-polymerization initiator is used. When a hybrid polymerization method (a method of using both heat and light) is employed, both the thermal polymerization initiator and the photo-polymerization initiator may be used. However, even in the case of the photo-polymerization method, a certain amount of heat is generated by light irradiation such as ultraviolet irradiation, etc., and a certain amount of heat is generated according to the progression of the polymerization reaction, which is an exothermic reaction, and therefore, the thermal polymerization initiator may be additionally used.

The photo-polymerization initiator may be used without limitation in view of composition as long as it is a compound capable of forming a radical by light such as ultraviolet rays.

The photo-polymerization initiator may include, for example, one or more selected from the group consisting of benzoin ether, dialkyl acetophenone, hydroxyl alkylketone, phenyl glyoxylate, benzyl dimethyl ketal, acyl phosphine, and $\alpha$-aminoketone. Further, specific examples of the acyl phosphine may include diphenyl(2,4,6-trimethylbenzoyl)phosphine oxide, phenylbis(2,4,6-trimethylbenzoyl)phosphine oxide, ethyl(2,4,6-trimethylbenzoyl)phenylphosphinate, etc. More various photo-polymerization initiators are well disclosed in "UV Coatings: Basics, Recent Developments and New Application(Elsevier, 2007)" written by Reinhold Schwalm, p 115, however, the photo-polymerization initiator is not limited to the above-described examples.

Further, as the thermal polymerization initiator, one or more selected from the group consisting of a persulfate-based initiator, an azo-based initiator, hydrogen peroxide, and ascorbic acid may be used. Specific examples of the persulfate-based initiator may include sodium persulfate (NazSzOs), potassium persulfate ($K_2S_2O_8$), ammonium persulfate ($(NH_4)_2S_2O_8$), etc., and examples of the azo-based initiator may include 2,2-azobis-(2-amidinopropane)dihydrochloride, 2,2-azobis-(N,N-dimethylene)isobutyramidine dihydrochloride, 2-(carbamoylazo)isobutyronitrile, 2,2-azobis[2-(2-imidazolin-2-yl)propane]dihydrochloride, 4,4-azobis-(4-cyanovaleric acid), etc. More various thermal polymerization initiators are well disclosed in 'Principle of Polymerization (Wiley, 1981)' written by Odian, p 203, however, the thermal polymerization initiator is not limited to the above-described examples.

The polymerization initiator may be used in an amount of 2 parts by weight or less with respect to 100 parts by weight of the water-soluble ethylenically unsaturated monomer. In other words, when the concentration of the polymerization initiator is too low, the polymerization rate may become slow, and a large amount of monomers remaining in the final product may be extracted, which is not preferred. On the contrary, when the concentration of the polymerization initiator is higher than the above range, polymer chains constituting the network become short, and thus the content of water-soluble components is increased and physical properties of the polymer may deteriorate, such as a reduction in absorbency under pressure, which is not preferred.

The monomer composition may further include an additive such as a thickener, a plasticizer, a preservation stabilizer, an antioxidant, etc., as needed.

Further, the monomer composition including the monomer may be, for example, in a solution state, in which it is dissolved in a solvent such as water. The solid content of the monomer composition in the solution state, i.e., the concentration of the monomer, the internal crosslinking agent, and the polymerization initiator, may be appropriately controlled in consideration of the polymerization time, the reaction conditions, etc. For example, the solid content in the monomer composition may be 10% by weight to 80% by weight, or 15% by weight to 60% by weight, or 30% by weight to 50% by weight.

When the monomer composition has the solid content in the above range, it may be advantageous in controlling the pulverization efficiency during pulverization of the polymer to be described later while there is no need to remove unreacted monomers after polymerization by using a gel effect phenomenon that occurs in the polymerization reaction of an aqueous solution at a high concentration.

As the solvent to be applicable, any solvent may be used without limitations in view of composition as long as it is able to dissolve the above components. For example, one or more selected from water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethylether, toluene, xylene, butyrolactone, carbitol, methyl cellosolve acetate, and N,N-dimethylacetamide may be used in combination.

According to one embodiment of the present invention, the step of forming the polymer by performing polymerization of the monomer composition may be performed in a batch-type reactor.

In the common method of preparing the superabsorbent polymer composition, the polymerization method is largely classified into thermal polymerization and photo-polymerization according to a polymerization energy source. When the thermal polymerization is carried out, it may be carried out in a reactor like a kneader equipped with agitating spindles. When the photo-polymerization is carried out, it may be carried out in a reactor equipped with a movable conveyor belt or in a flat-bottomed vessel.

Meanwhile, in the polymerization method as described above, a polymer, of which molecular weight is not large and molecular weight distribution is wide, is generally formed according to a short polymerization reaction time (e.g., 1 hour or shorter).

Meanwhile, when the photo-polymerization is carried out in a reactor equipped with a movable conveyor belt or in a flat-bottomed vessel, the obtained water-containing gel polymer may be usually obtained as a sheet-like water-containing gel polymer having a width of the belt, and the thickness of the polymer sheet may vary depending on the concentration of the monomer composition fed thereto and the feeding speed or feeding amount, and usually, it is obtained at a thickness of about 0.5 cm to about 5 cm.

However, when the monomer composition is supplied to such an extent that the thickness of the sheet-like polymer becomes too thin, it is undesirable because the production efficiency is low, and when the thickness of the sheet-like polymer is thick for productivity, the polymerization reaction may not evenly occur over the entire thickness, and thus it is difficult to form a high-quality polymer.

In addition, in the polymerization in a reactor having agitating spindles, equipped with a conveyor belt, a new monomer composition is supplied to the reactor while the polymerization product moves, and thus polymerization continuously occurs, and polymers having different polymerization rates are mixed. Accordingly, uniform polymerization hardly occurs throughout the monomer composition, and overall physical properties may deteriorate.

In contrast, according to one embodiment of the present invention, as the polymerization is performed in a fixed-bed type in the batch-type reactor, there is little risk of mixing of polymers with different polymerization rates, and as a result, a polymer having uniform quality may be obtained.

In addition, the polymerization step is performed in the batch-type reactor having a predetermined volume, and it may be performed for a longer period of time, for example, 3 hours or longer than polymerization continuously performed in the reactor equipped with the conveyor belt. Despite the long polymerization time as described above, since the polymerization is performed for the water-soluble ethylenically unsaturated monomer in an unneutralized state, the monomer is not easily precipitated even after the long-time polymerization, and thus it is advantageous for the long-time polymerization.

Meanwhile, in the polymerization in the batch-type reactor, a thermal polymerization method is employed, and thus the thermal polymerization initiator among the above-described initiators may be used as the polymerization initiator.

Meanwhile, in one embodiment of the present invention, a reducing agent forming a redox couple with the initiator may be introduced to initiate the polymerization.

Specifically, when the initiator and the reducing agent are added to the polymer solution, they react with each other to form radicals.

The formed radicals react with the monomer. Since the oxidation-reduction reaction between the initiator and the reducing agent is highly reactive, the polymerization starts even though only small amounts of the initiator and the reducing agent are added, and thus there is no need to increase the process temperature. Accordingly, a low-temperature polymerization is possible, and changes in the physical properties of the polymer solution may be minimized.

The polymerization reaction using the oxidation-reduction reaction may smoothly occur even at a temperature near or below room temperature (25°C). For example, the polymerization reaction may be carried out at a temperature of 5°C or higher and 25°C or lower, or 5°C or higher and 20°C or lower.

In one embodiment of the present invention, when a persulfate-based polymerization initiator is used as the initiator, one or more selected from the group consisting of sodium metabisulfite (NazSzOs); tetramethyl ethylenediamine (TMEDA); a mixture of iron(II) sulfate and EDTA ($FeSO_4$/EDTA); sodium formaldehyde sulfoxylate; and disodium 2-hydroxy-2-sulfinoacetate may be used as the reducing agent.

For example, potassium persulfate is used as the initiator, and disodium 2-hydroxy-2-sulfinoacetate is used as the reducing agent; or ammonium persulfate is used as the initiator and tetramethylethylenediamine is used as the reducing agent; or sodium persulfate may be used as the initiator, and sodium formaldehyde sulfoxylate may be used as the reducing agent.

In another embodiment of the present invention, when a peroxide-based initiator is used as the initiator, one or more selected from the group consisting of ascorbic acid; sucrose; sodium sulfite ($Na_2SO_3$), sodium metabisulfite (NazSzOs); tetramethyl ethylenediamine (TMEDA); a mixture of iron(II) sulfate and EDTA ($FeSO_4$/EDTA); sodium formaldehyde sulfoxylate; disodium 2-hydroxy-2-sulfinoacteate; and disodium 2-hydroxy-2-sulfoacteate may be used as the reducing agent.

As the polymer obtained by such a method is polymerized using the ethylenically unsaturated monomer in the unneutralized state, a polymer having a high molecular weight and a uniform molecular weight distribution may be formed, as described above, and the content of water-soluble components may be reduced.

The polymer obtained by such a method may be in a water-containing gel state, and its water content may be 30% by weight to 80% by weight. For example, the water content of the polymer may be 30% by weight or more, or 45% by weight or more, or 50% by weight or more, and 80% by weight or less, or 70% by weight or less, or 60% by weight or less.

When the water content of the polymer is too low, the polymer may not be effectively pulverized because it is

difficult to secure an appropriate surface area in the subsequent pulverization step. When the water content of the polymer is too high, it is difficult to pulverize the polymer into the desired particle size, because the pressure applied in the subsequent pulverization step increases.

Meanwhile, throughout the present specification, the "water content" means a weight occupied by water with respect to the total weight of the polymer, which may be a value obtained by subtracting the weight of the dried polymer from the weight of the polymer. Specifically, the water content is defined as a value calculated by measuring the weight loss due to evaporation of moisture in the polymer during the process of drying by raising the temperature of the polymer in a crumb state through infrared heating. At this time, the drying conditions may be determined as follows: the drying temperature is increased from room temperature to about 180°C and then the temperature is maintained at 180°C, and the total drying time is set to 40 minutes, including 5 minutes for the temperature rising step.

**(Step 2 and Step 3)**

Next, the step of neutralizing at least part of the acidic groups of the polymer (step 2); and the step of preparing base polymer particles by micronizing the polymer in the presence of the additive represented by Chemical Formula 1 (step 3) are performed.

First, as the neutralizing agent used in the step 2, basic substances capable of neutralizing acidic groups, such as sodium hydroxide, potassium hydroxide, ammonium hydroxide, etc., may be used.

Further, the degree of neutralization, which refers to the degree of neutralization of the acidic groups included in the polymer by the neutralizing agent, may be 50 mol% to 90 mol%, or 60 mol% to 85 mol%, or 65 mol% to 85 mol%, or 65 mol% to 75 mol%. The range of the degree of neutralization may vary depending on the final physical properties. However, when the degree of neutralization is excessively high, absorbency of the superabsorbent polymer may decrease and the concentration of carboxyl groups on the surface of the particles is too low, and thus it is difficult to properly perform surface crosslinking in the subsequent process, and absorbency under pressure or liquid permeability may be reduced. On the contrary, when the degree of neutralization is excessively low, the absorbency of the polymer greatly deteriorates and the polymer may also exhibit hard-to-handle properties, such as those of an elastic rubber.

Next, the step of preparing base polymer particles by micronizing the polymer in the presence of the additive represented by Chemical Formula 1 (step 3) is performed.

Here, the step 2 and the step 3 may be performed sequentially, simultaneously, or alternately. In other words, the step of micronizing the polymer (step 3) is performed at the same time as the step 2, or before or after the step 2, in the presence of the surfactant.

The step is a step of micronizing the polymer in the presence of the additive of Chemical Formula 1, and is a step of performing fine cutting of the polymer into a size of several ten micrometers to several hundred micrometers and aggregation thereof at the same time, rather than chopping the polymer into a size of millimeters. In other words, the step is a step of preparing secondary aggregated particles, in which primary particles finely cut into a size of several ten micrometers to several hundred micrometers are aggregated, by providing appropriate adhesiveness for the polymer. The base polymer particles, which are secondary aggregated particles prepared by the step, may have a remarkably improved absorption rate due to greatly increased surface area while having a normal particle size distribution.

As described, after mixing the polymer and the additive of Chemical Formula 1, the polymer is micronized in the presence of the additive of Chemical Formula 1 to prepare the base polymer particles which are in the form of secondary aggregated particles, in which the mixture of the superabsorbent polymer particles and the surfactant are finely cut and aggregated.

With regard to the additive represented by Chemical Formula 1, those described in the above description of the superabsorbent polymer composition may also be applied.

Here, the "base polymer particles" are particles having a water content of about 30% by weight or more, and since the base polymer particles are those obtained by finely cutting and aggregating the polymer into particles without a drying process, they may have a water content of 30% by weight to 80% by weight, preferably, 70% by weight to 80% by weight, like the polymer.

Meanwhile, the additive of Chemical Formula 1 may be used in an amount of 0.01 part by weight to 10 parts by weight with respect to 100 parts by weight of the water-soluble ethylenically unsaturated monomer used in the crosslinking polymerization for the preparation of the polymer. When the additive of Chemical Formula 1 is used in an excessively small amount, it is not uniformly adsorbed onto the polymer surface, and thus re-aggregation of particles may occur after pulverization. When the surfactant is used in an excessively large amount, the overall physical properties of the superabsorbent polymer finally prepared may deteriorate. For example, the additive of Chemical Formula 1 may be used in an amount of 0.01 part by weight or more, 0.015 parts by weight or more, or 0.1 part by weight or more, and 5 parts by weight or less, 3 parts by weight or less, 2 parts by weight or less, or 1 part by weight or less with respect to 100 parts by weight of the water-soluble ethylenically unsaturated monomer.

The method of mixing the additive of Chemical Formula 1 with the polymer is not particularly limited as long as

it is a method capable of evenly mixing the surfactant with the polymer, and may be appropriately adopted and used. Specifically, the additive of Chemical Formula 1 may be mixed in a dry manner, or mixed in a solution state after being dissolved in a solvent, or the additive of Chemical Formula 1 may be melted and then mixed.

Among them, for example, the additive of Chemical Formula 1 may be mixed in a solution state after being dissolved in a solvent. In this regard, any kind of solvent may be used without any limitation on inorganic or organic solvents, but water is most appropriate in consideration of the ease of the drying process and the cost of the solvent recovery system. In addition, a method of placing the additive of Chemical Formula 1 and the polymer in a reaction tank and then mixing them, a method of placing the polymer in a mixer and then spraying the solution thereto, or a method of continuously supplying the polymer and the solution to a mixer which is continuously operated, and then mixing them may be used.

Meanwhile, according to one embodiment of the present invention, the step 2 and the step 3 may be performed sequentially, simultaneously, or alternately.

In other words, after neutralizing the acidic groups by adding a neutralizing agent to the polymer, the additive of Chemical Formula 1 may be added to the neutralized polymer to perform micronization of the polymer which is mixed with the additive, or the polymer may be subjected to neutralization and micronization by simultaneously adding the neutralizing agent and the additive to the polymer. Alternatively, the additive may be first added, and then the neutralizing agent may be added. Alternatively, the neutralizing agent and the additive may be alternately added. Alternatively, the additive may be first added to perform micronization, and then the neutralizing agent may be added to perform neutralization, and the additive may be further added to the neutralized water-containing gel polymer to further perform the micronization process.

Meanwhile, for even neutralization of the entire polymer, it may be preferable to have a predetermined time difference between the addition of the neutralizing agent and the micronization process.

At least part or a significant amount of the additive of Chemical Formula 1 may be present on the surface of the base polymer particles.

Here, "the additive of Chemical Formula 1 is present on the surface of the base polymer particles" means that at least part or a significant amount of the additive of Chemical Formula 1 is adsorbed or bound onto the surface of the base polymer particles. Specifically, the additive of Chemical Formula 1 may be physically or chemically adsorbed onto the surface of the base polymer particles. More specifically, the hydrophilic functional group of the additive of Chemical Formula 1 may be physically adsorbed onto the hydrophilic moiety of the surface of the base polymer particles by intermolecular forces such as dipole-dipole interaction. As described, the hydrophilic moiety of the additive of Chemical Formula 1 is physically adsorbed onto the surface of the base polymer particles to cover the surface, and the hydrophobic moiety of the additive of Chemical Formula 1 is not adsorbed onto the surface of the base polymer particles, and thus the base polymer particles may be coated with the additive of Chemical Formula 1 in a kind of micelle structure. This is because the additive of Chemical Formula 1 is not added during the polymerization process of the water-soluble ethylenically unsaturated monomer, but is added in the micronization step after the polymer is formed. The additive of Chemical Formula 1 may fully perform its role as the surfactant, and pulverization and aggregation occur at the same time to obtain particles with a large surface area by aggregation of fine particles, as compared to the case where the additive of Chemical Formula 1 is added during the polymerization process and the additive of Chemical Formula 1 is present inside the polymer.

According to one embodiment, the step of preparing the base polymer particles (step 3) may be performed using a micronizer.

The micronizer may include a body part including a transfer space through which the mixture of the polymer and the additive is transferred; a screw member which is rotatably installed inside the transfer space to transfer the mixture; a driving motor providing a rotational driving force for the screw member; a cutter member which is installed in the body part and includes perforated plates having a plurality of holes formed therein, and cuts the mixture while discharging the mixture to the outside of the body part.

According to one embodiment, the step 2 and the step 3 may be performed sequentially, simultaneously, or alternately, and may be performed using the micronizer.

In this case, the micronizer further includes neutralizing agent spraying nozzles installed inside the body part, and the neutralizing agent is sprayed through the neutralizing agent spraying nozzles, thereby performing the step 2 and the step 3 sequentially, simultaneously, or alternately.

Specifically, the neutralizing agent is injected into the body part through the neutralizing agent spraying nozzles to neutralize at least part of acidic groups of the polymer having the acidic groups in the mixture. Specifically, in the micronizer, the neutralizing agent is injected into the body part through the neutralizing agent spraying nozzles to neutralize at least part of acidic groups of polymers having the acidic groups in the mixture, and at the same time, the mixture is pulverized (micronized) while being discharged to the outside of the body part through the perforated plates.

Preferably, the neutralizing agent sprayed from the neutralizing agent spraying nozzles may be sprayed adjacent to the perforated plates, and in this case, when the neutralization process is performed, and at the same time, the mixture

is discharged through the holes of the perforated plates, it preferably serves as a slip agent in the mixture to reduce the load of the holes.

Preferably, the cutter member of the micronizer includes the perforated plates and cutting knives disposed adjacent to the perforated plates and disposed on the outlet side of the body part. The mixture passes through the perforated plates, and then the mixture is more effectively pulverized and micronized by the cutting knife.

In the micronizer, the cutter member may include a plurality of perforated plates and a plurality of cutting knives. The order of arrangement of the plurality of perforated plates and the plurality of cutting knives is not particularly limited, and they may be disposed sequentially, or disposed alternately with each other, or the plurality of perforated plates may be disposed consecutively, or the plurality of cutting knives may be disposed consecutively.

By including the plurality of perforated plates and cutting knives as described above, micronization may be performed a plurality of times in a single micronizer. Meanwhile, the plurality of neutralizing agent spraying nozzles may be disposed adjacent to any one or more of the plurality of perforated plates and cutting knives, and it is preferable that the neutralizing agent spraying nozzles are disposed adjacent to the perforated plates in terms of improving slip property.

In the micronizer, the size of holes formed in the perforated plate may be 0.1 mm to 30 mm, preferably, 0.5 mm to 25 mm, 1 mm to 20 mm, or 1 mm to 10 mm. When the perforated plate having the hole size is used, base polymer particles having a desired particle size may be prepared. As described above, when the cutter member includes a plurality of perforated plates, the size of holes formed in each of the perforated plates may satisfy the above range, and they may be the same as or different from each other.

According to one embodiment of the present invention, the step 3 may be performed a plurality of times, which is performed using a plurality of micronizers or a single micronizer including a plurality of perforated plates and/or a plurality of cutting knives. Alternatively, some of the plurality of micronizers may include a plurality of perforated plates and/or a plurality of cutting knives. The micronization step may be preferably performed once to 6 times or once to 4 times. When the step 3 is performed a plurality of times, the additive may be additionally injected a plurality of times.

The step of preparing base polymer particles by micronizing the polymer (step 3) may be performed such that the base polymer particles are micronized to an average particle size of 50 $\mu$m to 600 $\mu$m, preferably, 100 $\mu$m to 500 $\mu$m, 150 $\mu$m to 450 $\mu$m, or 200 $\mu$m to 400 $\mu$m. By satisfying the above particle size range, when the polymer is prepared as secondary particles in the form of aggregates of primary particles, and then subjected to the pulverizing and drying processes under milder conditions, the amount of fine particles generated during the process may be remarkably reduced.

As used herein, the average particle size "Dn" means a particle size or a particle diameter at a point where the cumulative distribution of the number of particles according to the particle size is n %. In other words, D50 represents the particle size at a point where the cumulative distribution of the number of particles according to the particle size is 50%, D90 represents the particle size at a point where the cumulative distribution of the number of particles according to the particle size is 90%, and D10 represents the particle size at a point where the cumulative distribution of the number of particles according to the particle size is 10%. The Dn may be measured using a laser diffraction method. Specifically, after the powder to be measured is dispersed in a dispersion medium, it is introduced into a commercially available laser diffraction particle size measuring device (e.g., Microtrac S3500) to measure the difference in the diffraction pattern according to the particle size when the particles pass through a laser beam, thereby calculating the particle size distribution. D10, D50, and D90 may be measured by calculating the particle sizes at points where the cumulative distribution of the number of particles according to the particle size in the measuring device is 10%, 50%, and 90%.

**(Step 4)**

Next, the step of drying the base polymer particles (step 4) is included. Through this step, the superabsorbent polymer composition including the superabsorbent polymer particles and the additive represented by Chemical Formula 1 may be prepared.

The drying may be performed such that a plurality of superabsorbent polymer particles included in the prepared superabsorbent polymer composition have a water content of about 10% by weight or less, specifically, about 0.1% by weight to about 10% by weight, respectively.

At this time, drying of the dry product may be performed in a moving-type. The moving-type drying is distinguished from a fixed-bed type drying according to whether or not materials flow during drying.

In the common method of preparing superabsorbent polymers, the drying step is generally performed until the water content of the superabsorbent polymer particles is less than 10% by weight. In contrast, in the present invention, aggregation is controlled by the step of performing micronization in the presence of the additive of Chemical Formula 1, and thus drying is performed so that the water content of the dried superabsorbent polymer particles is 10% by weight to 20% by weight, preferably 10%% by weight to 25% by weight. However, the present invention is not limited thereto. Accordingly, there is an advantage in that generation of fine particles may be basically prevented due to the high water content. Further, the absorption rate of the final superabsorbent polymer composition may be preferably improved.

To this end, the drying step is performed at a relatively low temperature in a moving-type. The moving-type

drying is distinguished from a fixed-bed type drying according to whether or not materials flow during drying, and preferably prevents the aggregation phenomenon between the finely cut water-containing superabsorbent polymer particles in the pulverized product to be dried and completes drying within a short time.

To this end, the drying step is performed at a relatively low temperature in a moving-type. The moving-type drying is distinguished from the fixed-bed type drying according to whether or not materials flow during drying, and preferably prevents the aggregation phenomenon between the finely cut base polymer particles in the pulverized product to be dried and completes drying within a short time.

Specifically, the moving-type drying refers to a method of drying a pulverized product under mechanical stirring. At this time, the direction in which the hot air passes through the material may be the same as or different from the circulation direction of the material. Alternatively, the material may be dried by circulating the material inside the dryer, and passing a heating medium fluid (heating medium oil) through a separate pipe outside the dryer. In contrast, the fixed-bed type drying refers to a method in which a material to be dried is fixed on a floor such as a perforated iron plate through which air can pass, and is dried by passing hot air through the material from the bottom to top.

In the step of drying the base polymer particles (step 4), moving-type dryers generally used may be used without particular limitation. For example, the step may be performed using a moving-type dryer such as a horizontal-type mixer, a rotary kiln, a paddle dryer, or a steam tube dryer.

The drying step (step 4) may be performed at a relatively low temperature of 150°C or less, preferably, at 100°C to 150°C, 100°C to 130°C, 105°C to 115°C, and even when the drying step is performed at a low temperature as described above, superabsorbent polymer particles having the desired particle size and physical properties may be prepared without aggregation.

Meanwhile, the drying temperature may be an internal operating temperature of the moving-type dryer, into which the pulverized product is injected, and the drying temperature may be controlled by passing a heating medium fluid (heating medium oil) through a separate pipe outside the dryer, but is not limited thereto.

The drying step (step 4) may be performed for 30 minutes to 80 minutes, and may be performed for 30 minutes to 60 minutes, or for 40 minutes to 50 minutes. Since the aggregation phenomenon between the finely cut polymer resin particles in the pulverized product to be dried is small, even though the drying step is performed for a short time at a relatively low temperature, superabsorbent polymer particles having the desired particle size and physical properties may be prepared.

**(Additional step)**

Thereafter, the method of preparing the superabsorbent polymer composition according to one embodiment of the present invention may further include the steps of pulverizing and classifying the superabsorbent polymer particles, as needed.

Specifically, the pulverizing step may be performed such that the dry superabsorbent polymer particles are pulverized to have a particle size of normal particles, i.e., a particle size of 150 μm to 850 μm.

A pulverizer used therefor may specifically include a vertical pulverizer, a turbo cutter, a turbo grinder, a rotary cutter mill, a cutter mill, a disc mill, a shred crusher, a crusher, a chopper, or a disc cutter, etc., but is not limited to the above-described examples.

Alternatively, as the pulverizer, a pin mill, a hammer mill, a screw mill, a roll mill, a disc mill, a jog mill, etc. may be used, but is not limited to the above-described examples.

On the other hand, in the preparation method of the present invention, it is possible to realize the superabsorbent polymer particles having a narrower particle size distribution than those in the traditional chopping step, through the micronizing step, and when the moving-type drying is performed, the water content after drying is maintained as relatively high as 10% by weight or more. Thus, even though the pulverization is performed under mild conditions with a lower pulverization power, a superabsorbent polymer having a very high content of normal particles of 150 μm to 850 μm may be formed, and the generation rate of fine particles may be greatly reduced.

The superabsorbent polymer particles prepared as above may include superabsorbent polymer particles having a particle size of 150 μm to 850 μm, i.e., normal particles in an amount of 80% by weight or more, 85% by weight or more, 89% by weight or more, 90% by weight or more, 92% by weight or more, 93% by weight or more, 94% by weight or more, or 95% by weight or more with respect to the total weight. The above particle size of the polymer particles may be measured according to the European Disposables and Nonwovens Association (EDANA) standard WSP 220.3 method.

Further, the superabsorbent polymer particles may include fine particles having a particle size of less than 150 μm in an amount of less than about 20% by weight, less than about 18% by weight, less than about 15% by weight, less than about 13% by weight, less than about 10% by weight, specifically, less than about 5% by weight, more specifically, less than about 3% with respect to the total weight. This is in contrast to the traditional preparation method of the superabsorbent polymer, in which fine particles of more than about 20% by weight to about 30% by weight are

generated.

Next, the method of preparing the superabsorbent polymer according to one embodiment of the present invention may include the step of preparing superabsorbent polymer particles by thermal crosslinking of the surface of the base polymer particles in the presence of a surface crosslinking agent.

The surface crosslinking step is to induce a crosslinking reaction on the surface of the base polymer powder in the presence of the surface crosslinking agent, and unsaturated bonds of the water-soluble ethylenically unsaturated monomers, which remain uncrosslinked on the surface, may be crosslinked by the surface crosslinking agent, and as a result, the superabsorbent polymer having an increased surface crosslinking density may be formed.

Specifically, a surface-crosslinked layer may be formed by the heat treatment process in the presence of the surface crosslinking agent, and the heat treatment process increases the surface crosslinking density, i.e., the external crosslinking density, whereas the internal crosslinking density does not change. The superabsorbent polymer, in which the surface-crosslinked layer is formed, may have a structure in which the external crosslinking density is higher than the internal crosslinking density.

The surface crosslinking process may be performed at a temperature of 80°C to 250°C. More specifically, the surface crosslinking process may be performed at a temperature of 100°C to 220°C, or 120°C to 200°C for about 20 minutes to about 2 hours, or about 40 minutes to about 80 minutes. When satisfying the above-described conditions of the surface crosslinking process, the surface of the superabsorbent polymer particles may be sufficiently crosslinked to increase absorbency under pressure.

By satisfying these surface crosslinking process conditions (particularly, heating conditions and reaction conditions at the maximum reaction temperature), it is possible to prepare a superabsorbent polymer that appropriately satisfies physical properties such as excellent absorption rate, etc.

A heating means for the surface crosslinking reaction is not particularly limited. Heating may be performed by providing a heating medium or by directly providing a heat source. In this regard, the kind of the applicable heating medium may be steam, hot air, a hot fluid such as hot oil or the like, but is not limited thereto. Further, the temperature of the heating medium to be provided may be properly selected in consideration of the means of the heating medium, the heating rate, and the heating target temperature. Meanwhile, as the heat source to be directly provided, an electric heating or gas heating method may be used, but is not limited to the above-described examples.

Meanwhile, as the surface crosslinking agent included in the surface crosslinking agent composition, any surface crosslinking agent that has been traditionally used for the preparation of superabsorbent polymers may be used without any particular limitation. For example, the surface crosslinking agent may include one or more polyols selected from the group consisting of ethylene glycol, propylene glycol, 1,3-propanediol, 1,4-butanediol, 1,6-hexanediol, 1,2-hexanediol, 1,3-hexanediol, 2-methyl-1,3-propanediol, 2,5-hexanediol, 2-methyl-1,3-pentanediol, 2-methyl-2,4-pentanediol, tripropylene glycol and glycerol; one or more carbonate-based compounds selected from the group consisting of ethylene carbonate and propylene carbonate; an epoxy compound such as ethylene glycol diglycidyl ether, etc.; an oxazoline compound such as oxazolidinone, etc.; a polyamine compound; an oxazoline compound; a mono-, di- or poly-oxazolidinone compound; a cyclic urea compound; etc. Preferably, those the same as the above-described internal crosslinking agent may be used, and for example, a diglycidyl ether-based compound of alkylene glycol, such as ethyleneglycol diglycidyl ether, etc., may be used.

In the surface crosslinking step, a surface crosslinking agent composition including an alcohol-based solvent and water, in addition to the surface crosslinking agent, may be used.

Such a surface crosslinking agent may be used in an amount of 0.001 part by weight to 2 parts by weight with respect to 100 parts by weight of the superabsorbent polymer particles. Preferably, the surface crosslinking agent may be used in an amount of 0.005 parts by weight or more, 0.01 parts by weight or more, or 0.02 parts by weight or more, and 0.5 parts by weight or less, 0.3 parts by weight or less. By controlling the content range of the surface crosslinking agent within the above-described range, it is possible to prepare a superabsorbent polymer exhibiting excellent overall physical properties, such as absorption performances, liquid permeability, etc.

Meanwhile, the surface crosslinking agent is added to the superabsorbent polymer particles in the form of a surface crosslinking agent composition including the same. There is no particular limitation on the method of adding the surface crosslinking agent composition. For example, a method of placing the surface crosslinking agent composition and the superabsorbent polymer particles in a reactor and mixing them, a method of spraying the surface crosslinking agent composition onto the superabsorbent polymer particles, a method of continuously supplying the superabsorbent polymer particles and the surface crosslinking agent composition to a mixer continuously operated and mixing them, etc. may be used.

The surface crosslinking agent composition may further include water and/or hydrophilic organic solvent as a medium. Therefore, it is advantageous in that the surface crosslinking agent may be evenly dispersed on the base polymer powder. At this time, the content of water and the hydrophilic organic solvent may be preferably controlled with respect to 100 parts by weight of the superabsorbent polymer particles in order to induce uniform dissolution/dispersion of the surface crosslinking agent, to prevent the aggregation phenomenon of the base polymer powder, and at the same

time, to optimize the surface penetration depth of the surface crosslinking agent.

Meanwhile, in the method of preparing the superabsorbent polymer according to one embodiment of the present invention, aluminum salts such as aluminum sulfate salts and other various polyvalent metal salts may be further used during surface crosslinking in order to further improve liquid permeability, etc. These polyvalent metal salts may be included on the surface crosslinked layer of the superabsorbent polymer finally prepared.

According to one embodiment of the present invention, after the step of forming the surface-crosslinked layer on at least part of the surface of the superabsorbent polymer particles, any one or more of a cooling step of cooling the superabsorbent polymer particles on which the surface-crosslinked layer is formed, a hydrating step of adding water to the superabsorbent polymer particles on which the surface-crosslinked layer is formed, and a post-treatment step of adding an additive to the superabsorbent polymer particles on which the surface-crosslinked layer is formed, may be further included. In this regard, the cooling step, the hydrating step, and the post-treatment step may be performed sequentially or simultaneously.

The additives injected in the post-treatment step may include a liquid permeability improver, an anti-caking agent, a fluidity improver, an antioxidant, etc., but the present invention is not limited thereto.

By selectively performing the cooling step, the hydrating step, and the post-treatment step, the water content of the final superabsorbent polymer may be improved, and a superabsorbent polymer article with higher quality may be manufactured.

According to another embodiment of the present invention, provided is a superabsorbent polymer composition prepared by the above preparation method.

The superabsorbent polymer composition prepared by the above preparation method may achieve a high water content without a separate additional hydrating process or an additive injecting process, and thus it is possible to provide a superabsorbent polymer having the low content of fine particles and having a water retention capacity (CRC) and absorbency under pressure (AUP), which are general absorption properties, equal to or higher than those of the superabsorbent polymer prepared by the traditional method while having excellent absorption rate due to the low content of water-soluble components (EC).

Hereinafter, the actions and effects of the present invention will be described in more detail with reference to specific exemplary embodiments of the present invention. However, these exemplary embodiments are provided only for illustrating the present invention, and the scope of the present invention is not limited thereto.

## Example - Preparation of superabsorbent polymer composition

### Example 1

(Step 1) In a 5L glass container equipped with a stirrer and a thermometer, 1,000 g of acrylic acid, 3.5 g of pentaerythritol triallyl ether as an internal crosslinking agent, and 2,260 g of water were mixed, and stirred while maintaining at 5°C. 1,000 cc/min of nitrogen was introduced into the glass container containing the mixture for 1 hour, and replaced with nitrogen conditions. Next, 13 g of a 0.3% hydrogen peroxide aqueous solution as a polymerization initiator, 15 g of a 1% ascorbic acid aqueous solution, and 30 g of a 2% 2,2'-azobis-(2-amidinopropane)dihydrochloride aqueous solution were added, and at the same time, 15 g of a 0.01% iron sulfate aqueous solution as a reducing agent were added to initiate polymerization. After the temperature of the mixture reached 85°C, polymerization was allowed at 90$\pm$2°C for about 3 hours to obtain a polymer. The water content of the polymer was about 70% by weight.

(Step 2 and Step 3) 1.19 g of Compound A-1 of Table 1 as the additive of Chemical Formula 1 was water-dispersed in 52.27 g of water (preparation of a 2 wt% aqueous dispersion), and then mixed with 1,000 g of the polymer obtained in the step 1.

A first micronization process was performed by passing the mixture once through a first micronizer equipped with perforated plates containing a number of holes with a hole size of 6 mm.

Next, second, third, and fourth micronization processes were performed by passing the mixture a total of three times through a second micronizer equipped with perforated plates containing a number of holes with a hole size of 4 mm.

In the second micronization process, 400 g of 32% NaOH aqueous solution was injected through neutralizing agent nozzles which were disposed adjacent to the perforated plates, and a neutralization processes was performed together with the micronization.

In the third micronization process, 37.5 g of 15% $Na_2SO_4$ aqueous solution was injected through neutralizing agent nozzles which were disposed adjacent to the perforated plates, and a neutralization processes was performed together with the micronization.

In the last fourth micronization process, the micronization process was performed without adding the neutralizing agent or surfactant to obtain base polymer particles.

The degree of neutralization of the base polymer particles was 70 mol%.

(Step 4) Then, 1,000 g of the base polymer particles were put into a rotary mixer moving-type dryer rotating at

100 rpm. Drying was performed for 60 minutes while maintaining the internal temperature of the dryer at 105°C, thereby obtaining superabsorbent polymer particles. The water content of the superabsorbent polymer particles was 11wt%.

**Example 2**

A superabsorbent polymer composition was prepared in the same manner as in Example 1, except that Compound A-2 of Table 1, instead of Compound A-1, was used as the additive of Chemical Formula 1 in Example 1.
After drying of the step 4, the water content of final superabsorbent polymer particles was 11 wt%.

**Example 3**

A superabsorbent polymer composition was prepared in the same manner as in Example 1, except that Compound A-3 of Table 1, instead of Compound A-1, was used as the additive of Chemical Formula 1 in Example 1.
After drying of the step 4, the water content of final superabsorbent polymer particles was 11 wt%.

**Example 4**

A superabsorbent polymer composition was prepared in the same manner as in Example 1, except that Compound A-4 of Table 1, instead of Compound A-1, was used as the additive of Chemical Formula 1 in Example 1.
After drying of the step 4, the water content of final superabsorbent polymer particles was 11 wt%.

**Example 5**

A superabsorbent polymer composition was prepared in the same manner as in Example 1, except that Compound A-5 of Table 1, instead of Compound A-1, was used as the additive of Chemical Formula 1 in Example 1.
After drying of the step 4, the water content of final superabsorbent polymer particles was 14 wt%.

**Example 6**

A superabsorbent polymer composition was prepared in the same manner as in Example 1, except that Compound A-6 of Table 1, instead of Compound A-1, was used as the additive of Chemical Formula 1 in Example 1.
After drying of the step 4, the water content of final superabsorbent polymer particles was 14 wt%.

**Example 7**

A superabsorbent polymer composition was prepared in the same manner as in Example 1, except that Compound A-7 of Table 1, instead of Compound A-1, was used as the additive of Chemical Formula 1 in Example 1.
After drying of the step 4, the water content of final superabsorbent polymer particles was 11 wt%.

**Example 8**

A superabsorbent polymer composition was prepared in the same manner as in Example 1, except that Compound A-8 of Table 1, instead of Compound A-1, was used as the additive of Chemical Formula 1 in Example 1.
After drying of the step 4, the water content of final superabsorbent polymer particles was 12 wt%.

**Example 9**

A superabsorbent polymer composition was prepared in the same manner as in Example 1, except that Compound A-9 of Table 1, instead of Compound A-1, was used as the additive of Chemical Formula 1 in Example 1.
After drying of the step 4, the water content of final superabsorbent polymer particles was 13 wt%.

**Example 10**

A superabsorbent polymer composition was prepared in the same manner as in Example 1, except that Compound A-10 of Table 1, instead of Compound A-1, was used as the additive of Chemical Formula 1 in Example 1.
After drying of the step 4, the water content of final superabsorbent polymer particles was 11 wt%.

**Example 11**

(Step 1) In a 5L glass container equipped with a stirrer and a thermometer, 1,000 g of acrylic acid, 3.5 g of pentaerythritol triallyl ether as an internal crosslinking agent, and 2,260 g of water were mixed, and stirred while maintaining at 5°C. 1,000 cc/min of nitrogen was introduced into the glass container containing the mixture for 1 hour, and replaced with nitrogen conditions. Next, 13 g of a 0.3% hydrogen peroxide aqueous solution as a polymerization initiator, 15 g of a 1% ascorbic acid aqueous solution, and 30 g of a 2% 2,2'-azobis-(2-amidinopropane)dihydrochloride aqueous solution were added, and at the same time, 15 g of a 0.01% iron sulfate aqueous solution as a reducing agent were added to initiate polymerization. After the temperature of the mixture reached 85°C, polymerization was allowed at 90$\pm$2°C for about 3 hours to obtain a polymer. The water content of the polymer was about 70% by weight.
(Step 2 and Step 3) 0.595 g of Compound A-1 of Table 1 as the additive of Chemical Formula 1 was water-dispersed in 52.27 g of water (preparation of a 2 wt% aqueous dispersion), and then mixed with 1,000 g of the polymer obtained in the step 1.
A first micronization process was performed by passing the mixture once through a first micronizer equipped with perforated plates containing a number of holes with a hole size of 6 mm.
Next, second, third, and fourth micronization processes were performed by passing the mixture a total of three times through a second micronizer equipped with perforated plates containing a number of holes with a hole size of 4 mm.
In the second micronization process, 400 g of 32% NaOH aqueous solution was injected through neutralizing agent nozzles which were disposed adjacent to the perforated plates, and a neutralization processes was performed together with the micronization.
In the third micronization process, 37.5 g of 15% $Na_2SO_4$ aqueous solution was injected through neutralizing agent nozzles which were disposed adjacent to the perforated plates, and a neutralization processes was performed together with the micronization.
In the last fourth micronization process, 0.595 g of Compound A-11 of Table 1 as the additive of Chemical Formula 1 was water-dispersed in 52.27 g of water (preparation of a 2 wt% aqueous dispersion), and injected to the second micronizer containing the mixture which was subjected to the third micronization, and the fourth micronization process was performed to prepare base polymer particles. The degree of neutralization of the base polymer particles was 70 mol%.
(Step 4) Then, 1,000 g of the base polymer particles were put into a rotary mixer moving-type dryer rotating at 100 rpm. Drying was performed for 60 minutes while maintaining the internal temperature of the dryer at 105°C, thereby obtaining superabsorbent polymer particles. After the step 4, the water content of the final superabsorbent polymer particles was 12wt%.

**Example 12**

A superabsorbent polymer composition was prepared in the same manner as in Example 11, except that Compound A-1 was used as the additive of Chemical Formula 1 in the first micronization step, and Compound A-2 of Table 1, instead of Compound A-11, was used as the additive of Chemical Formula 1 in the fourth micronization step in Example 11.
After drying of the step 4, the water content of final superabsorbent polymer particles was 10 wt%.

**Example 13**

A superabsorbent polymer composition was prepared in the same manner as in Example 1, except that Compound A-12 of Table 1, instead of Compound A-1, was used as the additive of Chemical Formula 1 in Example 1.
After drying of the step 4, the water content of final superabsorbent polymer particles was 14 wt%.

**Example 14**

A superabsorbent polymer composition was prepared in the same manner as in Example 1, except that Compound A-13 of Table 1, instead of Compound A-1, was used as the additive of Chemical Formula 1 in Example 1.
After drying of the step 4, the water content of final superabsorbent polymer particles was 14 wt%.

**Example 15**

A superabsorbent polymer composition was prepared in the same manner as in Example 1, except that 0.595 g of Compound A-12 used in Example 13 and 0.595 g of Compound A-13 used in Example 14 were mixed and a total of 1.19 g of the additive was used as the additive of Chemical Formula 1.

After drying of the step 4, the water content of final superabsorbent polymer particles was 13 wt%.

**Example 16**

A superabsorbent polymer composition was prepared in the same manner as in Example 13, except that the amount of A-12 was increased to 2.38 g as the additive of Chemical Formula 1 in Example 13.

After drying of the step 4, the water content of final superabsorbent polymer particles was 12 wt%.

**Comparative Example 1**

A superabsorbent polymer composition was prepared in the same manner as in Example 1, except that the additive of Chemical Formula 1 was not used in Example 1.

**Comparative Examples 2 to 6, 8 and 9**

Each superabsorbent polymer composition was prepared in the same manner, except that the additive described in Table 1 below was used as the additive of Chemical Formula 1 in Example 1.

**Comparative Example 7**

In a 3L glass container equipped with a stirrer and a thermometer, 100 g (1.388 mol) of acrylic acid, 0.16 g of polyethylene glycol diacrylate (Mn= 508) as an internal crosslinking agent, 0.008 g of diphenyl(2,4,6-trimethylbenzoyl)-phosphine oxide as a photopolymerization initiator, 0.12 g of sodium persulfate as a thermal polymerization initiator, and 123.5 g of a 32% caustic soda solution were mixed at room temperature to prepare a monomer composition (Degree of neutralization of acrylic acid: 70 mol%, solid content: 45% by weight).

Thereafter, the monomer composition was fed on a conveyor belt at a rate of 500 mL/min to 2000 mL/min, the belt of 10 cm in width and 2 m in length rotating at a speed of 50 cm/min. Then, at the same time as the feeding of the monomer composition, the polymerization reaction was allowed for 60 seconds by irradiating ultraviolet rays with an intensity of 10 $mW/cm^2$, thereby obtaining a water-containing gel polymer having a water content of 55% by weight.

Next, the water-containing gel polymer obtained through the polymerization reaction was pulverized without the additive using a meat chopper. At this time, the water content of the water-containing superabsorbent polymer particles included in the final pulverized product was 55 wt%.

Next, the pulverized product was dried using a convection oven capable of shifting airflow up and down by flowing hot air at 185°C from the bottom to the top for 20 minutes and from the top to the bottom for 20 minutes, and as a result, a superabsorbent polymer, in which the water content of final superabsorbent polymer particles after drying was 25 wt%, was prepared.

[Table 1]

| Section | Additive of step 3 |
| --- | --- |
| Example 1 | 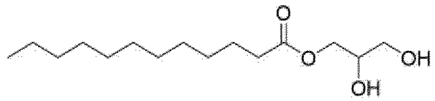 (A-1) |
| Example 2 | 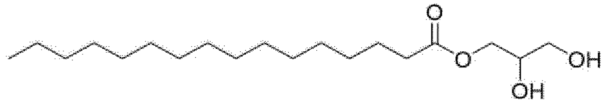 (A-2) |
| Example 3 | 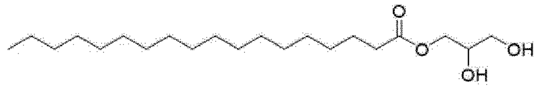 (A-3) |
| Example 4 | 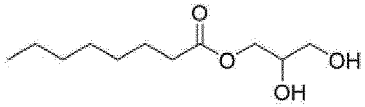 (A-4) |
| Example 5 | 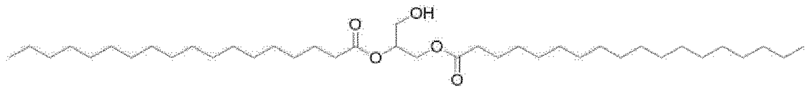 (A-5) |
| Example 6 | 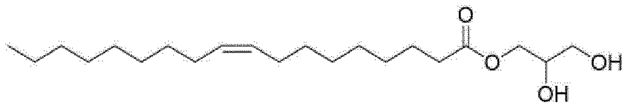 (A-6) |
| Example 7 | 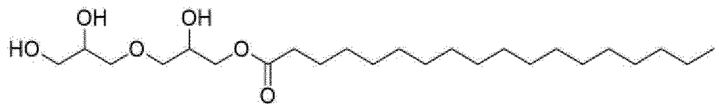 (A-7) |
| Example 8 | 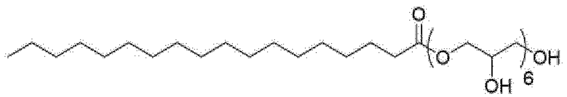 (A-8) |

(continued)

| Section | Additive of step 3 |
|---|---|
| Example 9 | 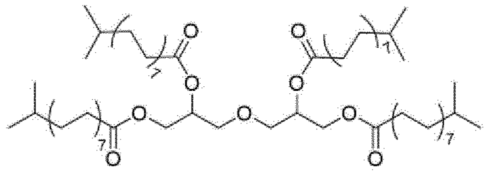 (A-9) |
| Example 10 | 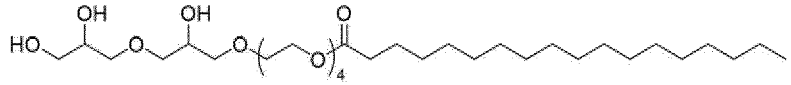 (A-10) |
| Example 11 | (A-1: first micronization)+ 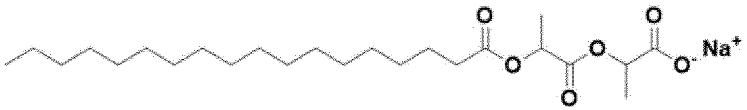 (A-11: fourth micronization) |
| Example 12 | (A-1: first micronization)+(A-2: fourth micronization) |
| Example 13 | 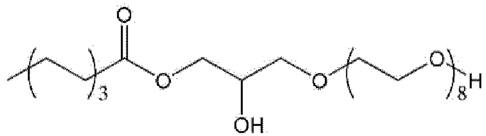 (A-12) 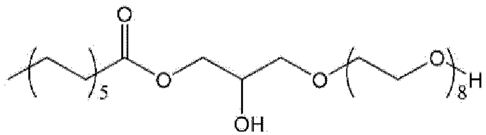 (A-13) |
| Example 14 | |
| Example 15 | (A-12: first micronization)+(A-13: first micronization) |
| Example 16 | (A-12) |
| Comparative Example 1 | Use of additive X |
| Comparative Example 2 | Monobutyl Maleate |

(continued)

| Section | Additive of step 3 |
|---|---|
| Comparative Example 3 | Monolauryl Glutarate |
| Comparative Example 4 | Dodecanoic Acid |
| Comparative Example 5 | Stearic Acid |
| Comparative Example 6 | Pluronic L35 |
| Comparative Example 7 | Use of additive X (prior-neutralization process) |
| Comparative Example 8 | laureth-3-adipate |
| Comparative Example 9 | Polyethyleneglycol Monomethacrylate (bisomer PEM6LD) 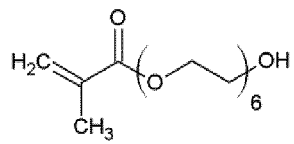 |

**Experimental Example**

Particle aggregation property, centrifuge retention capacity (CRC), absorbency under pressure (AUP), absorption rate, and effective absorbency were measured for the superabsorbent polymer compositions prepared in Examples and Comparative Examples by the following methods, respectively, and the results are shown in Tables 3 and 4, below. Unless otherwise indicated, the following evaluation of physical properties were all performed at constant temperature and constant humidity (23±2°C, relative humidity of 45±10%). In order to prevent measurement error, the average value of three measurements was used as measurement data. Further, physiological saline or brine used in the following evaluation of physical properties means 0.9% by weight of an aqueous sodium chloride (NaCl) solution.

(1) Evaluation of particle aggregation property

① 1,000 g of the unneutralized polymer (water content of 70wt%) of the step 1 prepared in each of Examples and Comparative Examples was prepared.
② Next, 0.36 g of the additive of Chemical Formula 1 or a corresponding comparative compound were water-dispersed in 17.54 g of water at 80°C (preparation of 2.04 wt% aqueous dispersion), which was mixed with 300 g of the polymer obtained in the step 1.

The additive of the step 2 or a corresponding comparative compound was mixed in the form of an aqueous solution according to the kind and content used in each Example and Comparative Example.

② 300 g of the mixture was put into a mincer including perforated plates with 3 mmΨ holes and 5T thickness, followed by pulverizing.
(1) For the pulverized product, the discharge time, discharge amount, and visual evaluation for aggregation according to evaluation criteria in Table 2 below were performed, and the results are shown in Table 3.

At this time, the discharge time means the time (seconds) taken for 200 g of the pulverized product to be discharged from the mincer, and the discharge time is shortened as the aggregation is alleviated.
The discharge amount means the ratio (wt%) of the amount discharged through the discharge port with respect to 300 g of the injected mixture, after the step ② is completed, and as the aggregation is alleviated, the amount of lumps remaining inside the mincer decreases, and thus the discharge amount increases.
In addition, photographs which are examples of the evaluation criteria ◦ and X of the visual evaluation for aggregation are shown in FIGS. 2A and 2B, respectively.

[Table 2]

| Evaluation | Criteria |
|---|---|
| X | 80% or more of the discharged product is discharged in the shape of a strand of 1 cm or more (see FIG. 2A). Due to the high adhesion to the blade, the polymer is crushed during chopping, and as a result, the surface of particles is uneven, and 80% or more of the discharged product has a strand shape of 1 cm or more due to high cohesiveness. |
| ○ | 80% or more of the discharged product is discharged in the shape of particles of less than 1 cm (see FIG. 2B). The adhesion to the blade and cohesiveness are greatly alleviated, and the surface of the particles after chopping is even, and 80% or more of the discharged product has a shape close to a spherical shape of less than 1 cm. |

[Table 3]

| | Kind of additive | Evaluation of particle aggregation property | | |
|---|---|---|---|---|
| | | Discharge time (sec) | Discharge amount (wt%) | Visual evaluation for aggregation |
| Experimental Example 1-1 | A-1 | 24 | 97.3 | ○ |
| Experimental Example 1-2 | A-2 | 55 | 95.6 | ○ |
| Experimental Example 1-3 | A-3 | 60 | 93.3 | ○ |
| Experimental Example 1-4 | A-4 | 61 | 92.6 | ○ |
| Experimental Example 1-5 | A-5 | 185 | 68.3 | ○ |
| Experimental Example 1-6 | A-6 | 165 | 71.6 | ○ |
| Experimental Example 1-7 | A-7 | 23 | 99.3 | ○ |
| Experimental Example 1-8 | A-8 | 184 | 72.6 | ○ |
| Experimental Example 1-9 | A-9 | 108 | 79.0 | ○ |
| Experimental Example 1-10 | A-10 | 35 | 97.3 | ○ |
| Experimental Example 1-11 | A-12 | 180 | 69.4 | ○ |
| Experimental Example 1-12 | A-13 | 178 | 70.1 | ○ |
| Comparative Experimental Example 1-1 | Without additive | More than 300 sec | 30.6 | X |
| Comparative Experimental Example 1-2 | Monobutyl Maleate | More than 300 sec | 34 | X |
| Comparative Experimental Example 1-3 | Monolauryl Glutarate | More than 300 sec | 34.6 | X |
| Comparative Experimental Example 1-4 | Dodecanoic Acid | More than 300 sec | 31 | X |
| Comparative Experimental Example 1-5 | Stearic Acid | More than 300 sec | 30.3 | X |
| Comparative Experimental Example 1-6 | Pluronic L35 | More than 300 sec | 37 | X |

(continued)

| | Kind of additive | Evaluation of particle aggregation property | | |
|---|---|---|---|---|
| | | Discharge time (sec) | Discharge amount (wt%) | Visual evaluation for aggregation |
| Comparative Experimental Example 1-8 | laureth-3-adipate | More than 300 sec | 35 | X |
| Comparative Experimental Example 1-9 | Polyethyleneglycol Monomethacrylate (bisomer PEM6LD) | More than 300 sec | 25 | X |

Referring to Table 3 and FIG. 2, it was confirmed that when pulverization was performed by adding the unneutralized polymer and the additive of Chemical Formula 1 of the present invention, aggregation between the particles after pulverization was suppressed, and the discharge time was short, and the discharge amount was remarkable, as compared to the case where the additive was not used or a compound not corresponding to the structure was used. It was also confirmed that the adhesion and cohesiveness of the mixtures introduced into the mincer were alleviated by the additive of Chemical Formula 1 of the present invention, and thus 80% or more of the discharged product was discharged in the form of particles of 1 cm, which was confirmed during visual evaluation.

(2) Centrifuge Retention Capacity (CRC)

The centrifuge retention capacity (CRC) by absorbency under no load was measured for each polymer composition in accordance with European Disposables and Nonwovens Association (EDANA) standard EDANA WSP 241.3.
In detail, from the superabsorbent polymer compositions obtained through Examples and Comparative Examples, polymers classified with #30-50 sieve were obtained. The polymer $W_0$(g) (about 0.2 g) was evenly put in a nonwoven fabric-made bag, followed by sealing. Then, the bag was immersed in a physiological saline solution (0.9 wt %) at room temperature. After 30 minutes, water was removed from the bag using a centrifuge at 250 G for 3 minutes, and the weight $W_2$(g) of the bag was then measured. Further, the same procedure was carried out without using the polymer, and then the resultant weight $W_1$(g) was measured.
By using the respective weights thus obtained, CRC (g/g) was calculated according to the following Equation 1, and the results are shown in Table 4.

[Equation 1]

$$\text{CRC (g/g)} = \{[W_2(g) - W_1(g)]/W_0(g)\} - 1$$

(3) Absorbency under Pressure (AUP)

Absorbency under pressure of 0.7 psi of the superabsorbent polymer compositions of Examples and Comparative Examples was measured in accordance with the EDANA method WSP 242.3.
First, when measuring absorbency under pressure, the classified polymer used at the time of CRC measurement was used.
In detail, a 400 mesh stainless steel net was installed in the bottom of a plastic cylinder having an internal diameter of 25 mm. The superabsorbent polymer composition $W_0$(g) (0.16 g) was uniformly scattered on the stainless steel net under conditions of room temperature and humidity of 50%. A piston capable of uniformly providing a load of 0.7 psi was placed thereon, in which an external diameter of the piston was slightly smaller than 25 mm, there was no gab between the internal wall of the cylinder and the piston, and the jig-jog of the cylinder was not interrupted. At this time, the weight $W_3$(g) of the apparatus was measured.
After placing a glass filter having a diameter of 90 mm and a thickness of 5 mm in a petri dish having a diameter of 150 mm, a physiological saline solution consisting of 0.9% by weight of sodium chloride was poured until the surface level of the physiological saline solution became equal to the upper surface of the glass filter. A sheet of filter paper having a diameter of 90 mm was placed on the glass filter. The measurement apparatus was mounted on the filter paper, thereby getting the liquid absorbed under the load for 1 hour. 1 hour later, the weight $W_4$(g) was measured after lifting

the measurement apparatus up.

Absorbency under pressure (g/g) was calculated from the obtained weights according to the following Equation 2, and the results are shown in Table 4.

[Equation 2]

$$AUP(g/g) = [W_4(g) - W_3(g)]/W_0(g)$$

(4) Surface tension (S/T)

In order to measure the surface tension of the superabsorbent polymer compositions of Examples and Comparative Examples, 0.5 g of each superabsorbent polymer composition was added to 40 mL of 0.9% saline, and stirred at 350 rpm for 3 minutes. After stopping the stirring, brine containing the swollen superabsorbent polymer was obtained. Using the brine as a measurement sample, the surface tension of each superabsorbent polymer composition was measured with a surface tension meter (product name: Force Tensiometer-K100, manufactured by KRUSS), and the results are shown in Table 4.

(5) Bulk density (BD)

100 g of each superabsorbent polymer composition of Examples and Comparative examples flowed through an orifice of a standard fluidity measuring device and placed in a container with a volume of 100 ml. Thereafter, the superabsorbent polymer composition was cut so as to be horizontal, and the volume of the superabsorbent polymer composition was adjusted to 100 ml. Then, the weight of only the superabsorbent polymer composition, excluding the container, was measured. The weight of only the superabsorbent polymer composition was then divided by 100 ml, which is the volume of the superabsorbent polymer composition, thereby obtaining the bulk density corresponding to the weight of the superabsorbent polymer composition per unit volume.

(6) Amount of fine particles generated

The amount of fine particles generated in each superabsorbent polymer composition prepared in Examples and Comparative Examples was calculated as a ratio of the weight of the polymer having a particle size of less than 150 μm with respect to the total weight after passing the prepared superabsorbent polymer composition through a coarse pulverizer (2800 rpm, 0.4 mm clearance, 1 mm lower mesh condition) once, and the results are shown in Table 4.

(7) Absorption rate (Vortex time)

The absorption rate (vortex time) of the superabsorbent polymer compositions of Examples and Comparative Examples was measured by the following method, and the results are shown in Table 4.

First, 50 mL of 0.9% brine was put in a 100 mL flat bottom beaker using a 100 mL mass cylinder.
② Next, the beaker was placed in the center of the magnetic stirrer, and then a magnetic bar (8 mm in diameter and 30 mm in length) was placed in the beaker.
Then, the stirrer was operated so that the magnetic bar stirred at 600 rpm, and the lowest part of the vortex generated by the stirring was allowed to touch the top of the magnetic bar.
④ After confirming that the temperature of the brine in the beaker reached 24.0°C, 2±0.01 g of the superabsorbent polymer composition sample was introduced, and at the same time, a stop watch was operated. The time taken for the vortex to disappear and for the liquid surface to be completely level was measured in second, and determined as the absorption rate.

[Table 4]

| Section | | Physical properties of SAP | | | | | |
|---|---|---|---|---|---|---|---|
| | | CRC (g/g) | AUP (g/g) | S/T (mN/m) | BD (g/ml) | Amount of fine particles generated (%) | Absorption rate (sec) |
| Experimental Example 2-1 | Example 1 | 43.5 | 27.4 | 71.5 | 0.72 | 1.4 | 23 |
| Experimental Example 2-2 | Example 2 | 42.5 | 27.3 | 71.1 | 0.71 | 2.3 | 25 |
| Experimental Example 2-3 | Example 3 | 42.3 | 26.5 | 71.2 | 0.71 | 2.7 | 26 |
| Experimental Example 2-4 | Example 4 | 42.1 | 27.3 | 71.3 | 0.72 | 4.8 | 30 |
| Experimental Example 2-5 | Example 5 | 41.6 | 27.0 | 71.2 | 0.71 | 3.5 | 29 |
| Experimental Example 2-6 | Example 6 | 41.4 | 26.9 | 71.3 | 0.71 | 3.2 | 29 |
| Experimental Example 2-7 | Example 7 | 42.4 | 27.4 | 71.4 | 0.72 | 4.3 | 28 |
| Experimental Example 2-8 | Example 8 | 42.0 | 27.0 | 71.0 | 0.70 | 5.2 | 28 |
| Experimental Example 2-9 | Example 9 | 41.8 | 26.8 | 71.1 | 0.70 | 3.0 | 27 |
| Experimental Example 2-10 | Example 10 | 42.2 | 27.4 | 70.9 | 0.70 | 1.9 | 24 |
| Experimental Example 2-11 | Example 11 | 43.4 | 27.5 | 71.2 | 0.71 | 1.1 | 22 |
| Experimental Example 2-12 | Example 12 | 42.6 | 26.8 | 71.0 | 0.71 | 1.2 | 21 |
| Experimental Example 2-13 | Example 13 | 40.5 | 24.3 | 71.0 | 0.70 | 4.4 | 30 |
| Experimental Example 2-14 | Example 14 | 41.8 | 25.3 | 71.1 | 0.71 | 2.5 | 28 |
| Experimental Example 2-15 | Example 15 | 40.9 | 24.5 | 71.1 | 0.71 | 4.0 | 30 |
| Experimental Example 2-16 | Example 16 | 42.1 | 25.8 | 71.1 | 0.71 | 3.1 | 28 |
| Comparative Experimental Example 2-1 | Comparative Example 1 | 37.7 | 25.5 | 71.1 | 0.69 | 14.5 | 38 |
| Comparative Experimental Example 2-2 | Comparative Example 2 | 38.3 | 25.0 | - | - | 15.1 | 35 |
| Comparative Experimental Example 2-3 | Comparative Example 3 | 36.8 | 25.7 | 68.9 | 0.68 | 16.2 | 36 |
| Comparative | Comparative Example | 37.0 | 25.5 | 68.1 | 0.68 | 16.3 | 36 |

(continued)

| Section | | Physical properties of SAP | | | | | |
|---|---|---|---|---|---|---|---|
| | | CRC (g/g) | AUP (g/g) | S/T (mN/m) | BD (g/ml) | Amount of fine particles generated (%) | Absorption rate (sec) |
| Experimental Example 2-4 | 4 | | | | | | |
| Comparative Experimental Example 2-5 | Comparative Example 5 | 37.8 | 25.3 | - | - | 14.9 | 35 |
| Comparative Experimental Example 2-6 | Comparative Example 6 | 38.5 | 25.0 | - | - | 11.7 | 35 |
| Comparative Experimental Example 2-7 | Comparative Example 7 | 36.7 | 24.3 | 71.3 | 0.68 | 15.4 | 40 |
| Comparative Experimental Example 2-8 | Comparative Example 8 | 38.5 | 25.1 | 70.5 | 0.69 | 13.5 | 34 |
| Comparative Experimental Example 2-9 | Comparative Example 9 | 35.9 | 24.5 | - | - | 16.7 | 42 |

Referring to Table 4, when the superabsorbent polymer composition was prepared by adding the unneutralized polymer and the additive of Chemical Formula 1 of the present invention, aggregation between particles after pulverization was suppressed, and it is possible to prepare the composition including the superabsorbent polymer particles of the desired particle size without the additional pulverization process after drying, and accordingly, the generation of fine particles was reduced, as compared to the case where the additive was not used, or the compound not corresponding to the structure was used, or the neutralized water-containing gel polymer was mixed. Accordingly, it was confirmed that the superabsorbent polymer compositions of Examples had a remarkably fast absorption rate, and exhibited the high bulk density without a decrease in the surface tension while having equivalent or higher water retention capacity and absorbency under pressure, as compared to those of Comparative Examples.

## Claims

**1.** A superabsorbent polymer composition comprising:

superabsorbent polymer particles including a crosslinked polymer of a water-soluble ethylenically unsaturated monomer having acidic groups and an internal crosslinking agent; and
an additive represented by the following Chemical Formula 1:

[Chemical Formula 1]

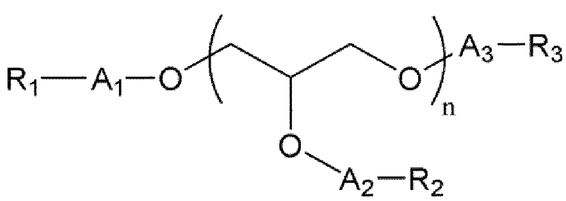

in Chemical Formula 1,

$A_1$, $A_2$, and $A_3$ are each independently a single bond, carbonyl,

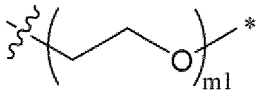

,

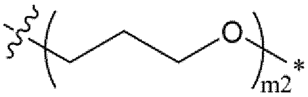

or

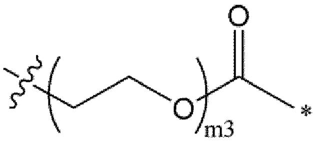

,

provided that one or more thereof are carbonyl or

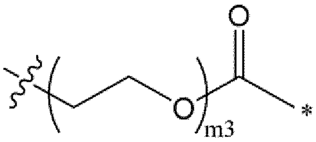

,

wherein m1, m2, and m3 are each independently an integer of 1 to 8,

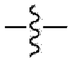

is connected to an adjacent oxygen atom,



is connected to adjacent $R_1$, $R_2$, and $R_3$, respectively,

$R_1$, $R_2$ and $R_3$ are each independently hydrogen, a straight or branched alkyl having 6 to 18 carbon atoms, or a straight or branched alkenyl having 6 to 18 carbon atoms, and

n is an integer of 1 to 9.

**2.** The superabsorbent polymer composition of claim 1, wherein the additive is one or more selected from compounds represented by the following Chemical Formula 1-1 to Chemical Formula 1-14:

[Chemical Formula 1-1]

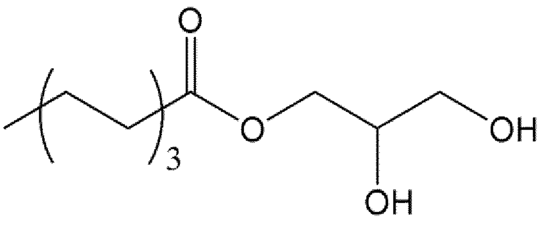

[Chemical Formula 1-2]

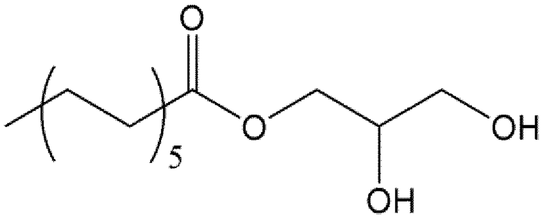

[Chemical Formula 1-3]

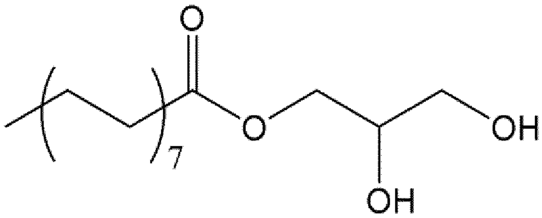

[Chemical Formula 1-4]

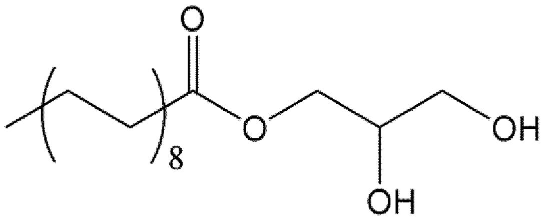

[Chemical Formula 1-5]

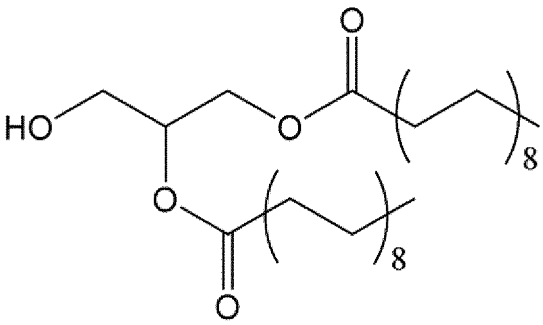

[Chemical Formula 1-6]

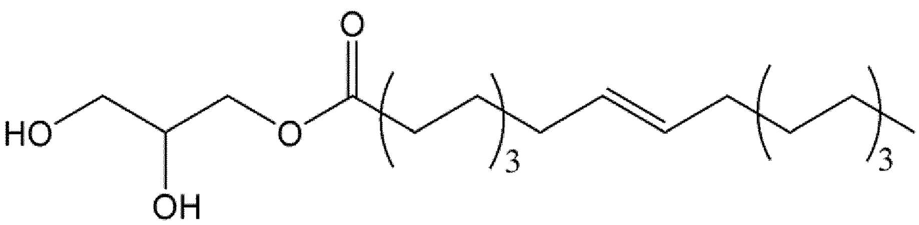

[Chemical Formula 1-7]

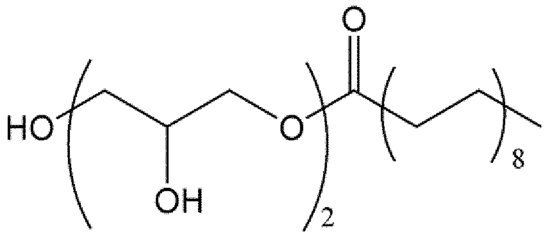

[Chemical Formula 1-8]

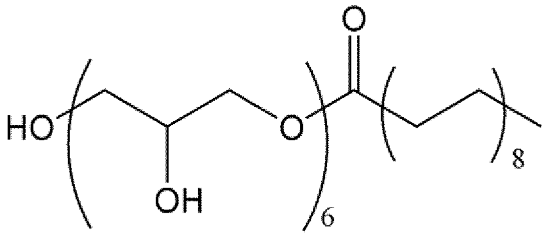

[Chemical Formula 1-9]

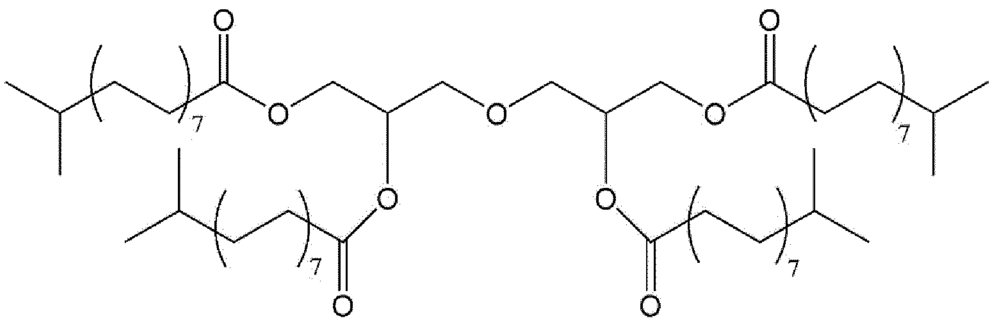

[Chemical Formula 1-10]

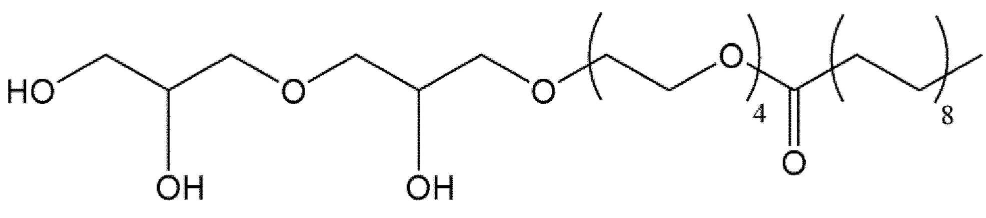

[Chemical Formula 1-11]

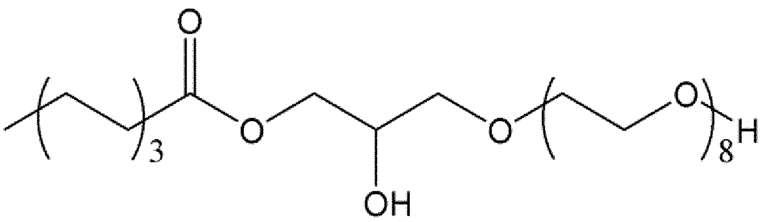

[Chemical Formula 1-12]

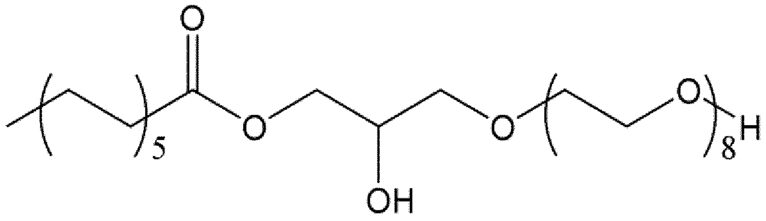

[Chemical Formula 1-13]

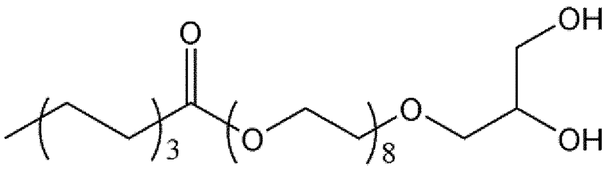

[Chemical Formula 1-14]

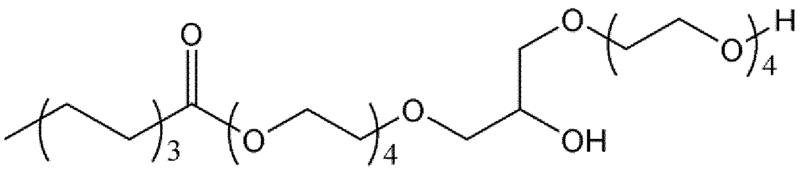

3. The superabsorbent polymer composition of claim 1, wherein the additive is included in an amount of 0.01 part by weight to 10 parts by weight with respect to 100 parts by weight of the water-soluble ethylenically unsaturated monomer.

4. The superabsorbent polymer composition of claim 1, further comprising a surface-crosslinked layer which is formed on at least part of the surface of the superabsorbent polymer particles by further crosslinking the crosslinked polymer via a surface crosslinking agent.

5. The superabsorbent polymer composition of claim 1, wherein the superabsorbent polymer composition has an absorption rate (vortex time) at 24.0°C of 30 seconds or less according to the vortex method.

6. The superabsorbent polymer composition of claim 1, wherein the superabsorbent polymer composition has a centrifuge retention capacity (CRC) of 39.0 g/g or more according to EDANA WSP 241.3.

7. The superabsorbent polymer composition of claim 1, wherein the superabsorbent polymer composition has absorbency under pressure (AUP) of 0.7 psi of 24.0 g/g or more according to the EDANA method WSP 242.3.

8. A method of preparing a superabsorbent polymer composition, the method comprising the steps of:

forming a polymer by crosslinking-polymerizing a water-soluble ethylenically unsaturated monomer having acidic groups, in the presence of an internal crosslinking agent and a polymerization initiator (step 1);
neutralizing at least part of the acidic groups of the polymer (step 2);
preparing base polymer particles by micronizing the polymer in the presence of an additive represented by the following Chemical Formula 1 (step 3); and
drying the base polymer particles (step 4):

[Chemical Formula 1]

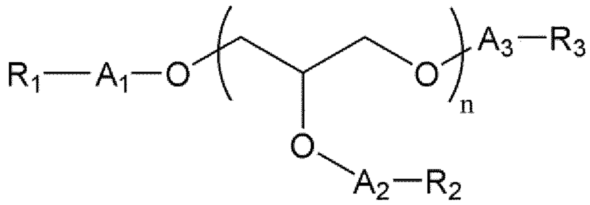

in Chemical Formula 1,
$A_1$, $A_2$, and $A_3$ are each independently a single bond, carbonyl,

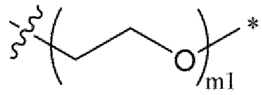

,

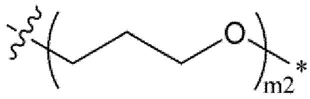

or

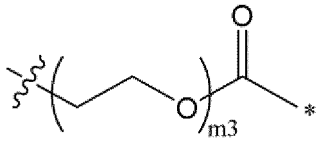

,

provided that one or more thereof are carbonyl or

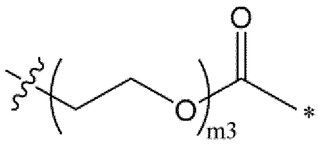

,

wherein m1, m2, and m3 are each independently an integer of 1 to 8,

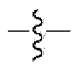

is connected to an adjacent oxygen atom,



is connected to adjacent $R_1$, $R_2$, and $R_3$, respectively,
$R_1$, $R_2$ and $R_3$ are each independently hydrogen, a straight or branched alkyl having 6 to 18 carbon atoms, or a straight or branched alkenyl having 6 to 18 carbon atoms, and
n is an integer of 1 to 9.

**9.** The method of claim 8, wherein the step of forming the polymer (step 1) is performed in a batch-type reactor.

**10.** The method of claim 8, wherein the step 2 and the step 3 are performed sequentially, simultaneously, or alternately.

**11.** The method of claim 8, wherein the additive is included in an amount of 0.01 part by weight to 10 parts by weight with respect to 100 parts by weight of the water-soluble ethylenically unsaturated monomer.

**12.** The method of claim 8, wherein the step of preparing the base polymer particles (step 3) is performed using a micronizer, the micronizer including:

a body part including a transfer space through which the mixture of the polymer and the additive is transferred;
a screw member which is rotatably installed inside the transfer space to transfer the mixture;
a driving motor providing a rotational driving force for the screw member; and
a cutter member which is installed in the body part and includes perforated plates having a plurality of holes formed therein, and cuts the mixture while discharging the mixture to the outside of the body part.

**13.** The method of claim 8, wherein the step 2 and the step 3 are performed sequentially, simultaneously, or alternately, and the step 2 and the step 3 are performed using a micronizer, the micronizer including:

a body part including a transfer space through which the mixture of the polymer and the additive is transferred;
a screw member which is rotatably installed inside the transfer space to transfer the mixture;
a driving motor providing a rotational driving force for the screw member;
a cutter member which is installed in the body part and includes perforated plates having a plurality of holes formed therein, and cuts the mixture while discharging the mixture to the outside of the body part; and
neutralizing agent spraying nozzles which are installed inside the body part.

**14.** The method of claim 13, wherein in the micronizer, the neutralizing agent is injected into the body part through the neutralizing agent spraying nozzles to neutralize at least part of acidic groups of the polymer having the acidic groups in the mixture.

**15.** The method of claim 12, wherein the size of holes formed in the perforated plates is 0.1 mm to 30 mm.

**16.** The method of claim 8, wherein the step of drying the base polymer particles (step 4) is performed in a moving-type.

**17.** The method of claim 8, wherein the step of drying the base polymer particles (step 4) is performed using a moving-type dryer of a horizontal-type mixer, a rotary kiln, a paddle dryer, or a steam tube dryer.

**18.** The method of claim 8, wherein the water content of the superabsorbent polymer particles obtained in the step of drying the base polymer particles (step 4) is 10% by weight to 20% by weight.

**19.** The method of claim 8, further comprising the step of forming a surface-crosslinked layer on at least part of the surface of the superabsorbent polymer particles prepared in the step 4, in the presence of a surface crosslinking agent.

【FIG. 1】

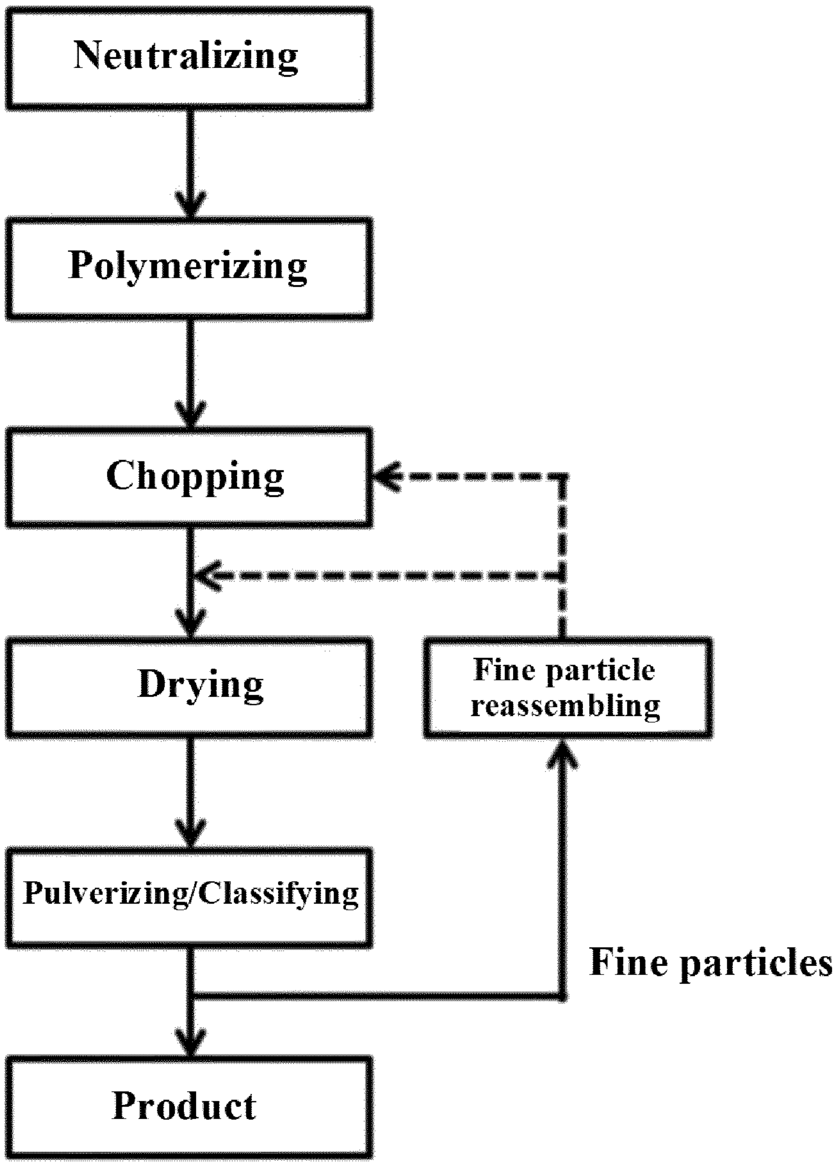
Neutralizing
Polymerizing
Chopping
Drying
Fine particle reassembling
Pulverizing/Classifying
Fine particles
Product

【FIG. 2】

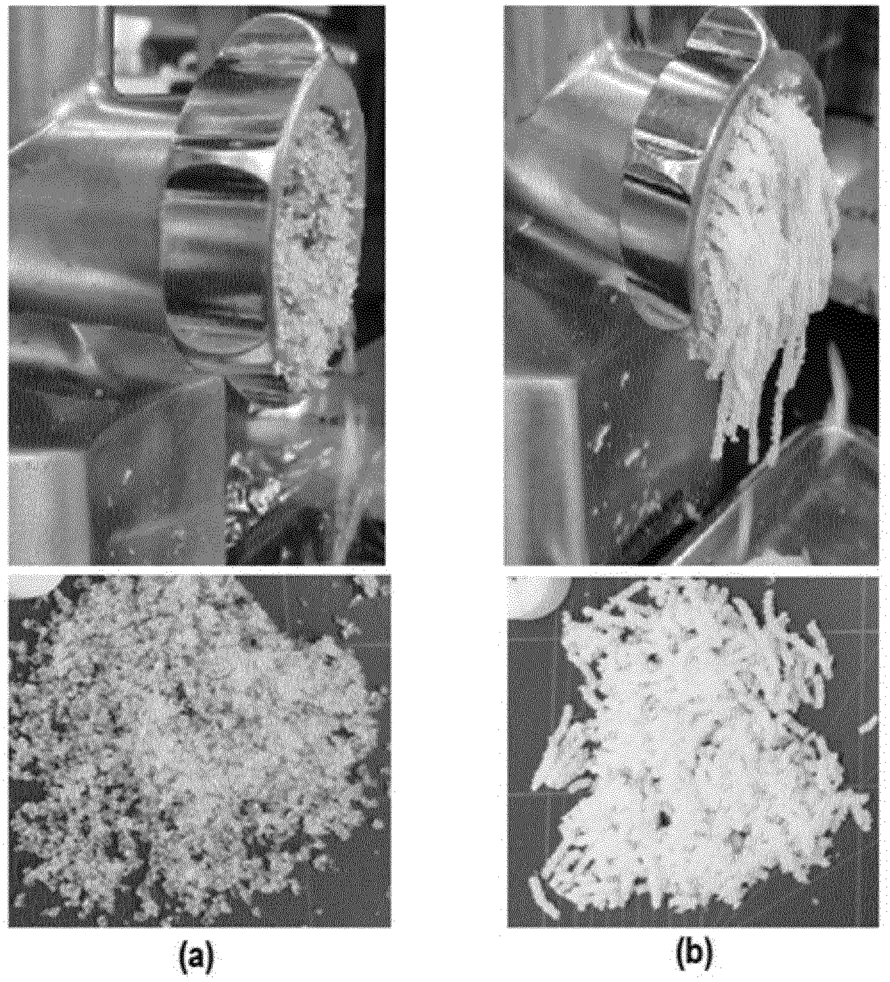

(a) (b)

**INTERNATIONAL SEARCH REPORT**

International application No.
**PCT/KR2022/016642**

**A. CLASSIFICATION OF SUBJECT MATTER**

**C08K 5/103**(2006.01)i; **C08L 33/06**(2006.01)i; **C08J 3/24**(2006.01)i; **C08J 3/12**(2006.01)i

According to International Patent Classification (IPC) or to both national classification and IPC

**B. FIELDS SEARCHED**

Minimum documentation searched (classification system followed by classification symbols)

C08K 5/103(2006.01); B02C 18/36(2006.01); B26D 5/22(2006.01); C08F 2/00(2006.01); C08F 2/44(2006.01); C08F 220/06(2006.01); C08F 6/06(2006.01); C08J 3/075(2006.01); C08J 3/12(2006.01); C08K 3/013(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 고흡수제(absorbent, super-absorbent, SAP), 첨가제(additives), 계면활성제(surfactant), 미립화(grind, atomize, pulverize, mill, crush), 중화(neutralization), 유동식 건조(moving type dry)

**C. DOCUMENTS CONSIDERED TO BE RELEVANT**

| Category* | Citation of document, with indication, where appropriate, of the relevant passages | Relevant to claim No. |
|---|---|---|
| X | KR 10-2019-0077541 A (NIPPON SHOKUBAI CO., LTD.) 03 July 2019 (2019-07-03)<br>See abstract; claims 1-3, 17-19, 23-25 and 35-39; paragraphs [0110], [0112], [0146]-[0152], [0180], [0239], [0263], [0264], [0278], [0338], [0401]-[0405], [0426], [0460], [0462] and [0476]; and figures 1 and 2. | 1-7 |
| Y | | 8-19 |
| Y | KR 10-2013-0096218 A (BASF SE) 29 August 2013 (2013-08-29)<br>See claim 1; and paragraphs [0105] and [0109]. | 8-19 |
| Y | KR 10-2021-0062459 A (LG CHEM, LTD.) 31 May 2021 (2021-05-31)<br>See claims 1 and 2; paragraphs [0054] and [0055]; and figure 3. | 12-15 |
| Y | JP 59-030826 A (CASSELLA FARBWERKE MAINKUR AG) 18 February 1984 (1984-02-18)<br>See claims 1-11; page 4; and the drawings. | 12-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 February 2023** | **03 February 2023** |
| Name and mailing address of the ISA/KR<br>**Korean Intellectual Property Office**<br>**Government Complex-Daejeon Building 4, 189 Cheongsa-ro, Seo-gu, Daejeon 35208** | Authorized officer |
| Facsimile No. **+82-42-481-8578** | Telephone No. |

Form PCT/ISA/210 (second sheet) (July 2022)

INTERNATIONAL SEARCH REPORT

International application No.
PCT/KR2022/016642

C. DOCUMENTS CONSIDERED TO BE RELEVANT

| Category* | Citation of document, with indication, where appropriate, of the relevant passages | Relevant to claim No. |
|---|---|---|
| A | JP 2016-124901 A (NIPPON SHOKUBAI CO., LTD.) 11 July 2016 (2016-07-11) See claims 1 and 6-8; and paragraphs [0037], [0085], [0086], [0093], [0094], [0128] and [0179]-[0197]. | 1-19 |
| A | KR 10-2020-0055648 A (LG CHEM, LTD.) 21 May 2020 (2020-05-21) See claims 1-14. | 1-19 |

Form PCT/ISA/210 (second sheet) (July 2022)

**INTERNATIONAL SEARCH REPORT**
**Information on patent family members**

International application No.
**PCT/KR2022/016642**

| Patent document cited in search report | Publication date (day/month/year) | Patent family member(s) | Publication date (day/month/year) |
|---|---|---|---|
| KR 10-2019-0077541 A | 03 July 2019 | CN 109996833 A | 09 July 2019 |
| | | CN 109996833 B | 11 February 2022 |
| | | CN 109996835 A | 09 July 2019 |
| | | CN 109996835 B | 29 July 2022 |
| | | EP 3543279 A1 | 25 September 2019 |
| | | EP 3543280 A1 | 25 September 2019 |
| | | JP 2019-052285 A | 04 April 2019 |
| | | JP 6800998 B2 | 16 December 2020 |
| | | JP 6913107 B2 | 04 August 2021 |
| | | JP 6918407 B2 | 11 August 2021 |
| | | KR 10-2019-0077540 A | 03 July 2019 |
| | | US 11465126 B2 | 11 October 2022 |
| | | US 2019-0329219 A1 | 31 October 2019 |
| | | US 2019-0329220 A1 | 31 October 2019 |
| | | WO 2018-092863 A1 | 24 May 2018 |
| | | WO 2018-092864 A1 | 24 May 2018 |
| KR 10-2013-0096218 A | 29 August 2013 | BR 112012031895 A2 | 08 November 2016 |
| | | CN 103068861 A | 24 April 2013 |
| | | CN 103068861 B | 25 November 2015 |
| | | EP 2580256 A2 | 17 April 2013 |
| | | JP 2013-530281 A | 25 July 2013 |
| | | JP 5766283 B2 | 19 August 2015 |
| | | WO 2011-157656 A2 | 22 December 2011 |
| | | WO 2011-157656 A3 | 26 July 2012 |
| KR 10-2021-0062459 A | 31 May 2021 | BR 112022001362 A2 | 22 March 2022 |
| | | CN 114096388 A | 25 February 2022 |
| | | EP 3984711 A1 | 20 April 2022 |
| | | JP 2022-541140 A | 22 September 2022 |
| | | US 2022-0258380 A1 | 18 August 2022 |
| | | WO 2021-101277 A1 | 27 May 2021 |
| JP 59-030826 A | 18 February 1984 | DE 000003221947 A1 | 22 December 1983 |
| | | EP 0096790 A1 | 28 December 1983 |
| | | EP 0096790 B1 | 16 December 1987 |
| | | JP 03-073576 B2 | 22 November 1991 |
| | | JP 0373576 B2 | 22 November 1991 |
| JP 2016-124901 A | 11 July 2016 | JP 6425341 B2 | 21 November 2018 |
| KR 10-2020-0055648 A | 21 May 2020 | CN 111436201 A | 21 July 2020 |
| | | EP 3680277 A1 | 15 July 2020 |
| | | JP 2021-510741 A | 30 April 2021 |
| | | JP 6973874 B2 | 01 December 2021 |
| | | KR 10-2418591 B1 | 07 July 2022 |
| | | US 11466131 B2 | 11 October 2022 |
| | | US 2021-0147640 A1 | 20 May 2021 |
| | | WO 2020-101167 A1 | 22 May 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210147025
- US 1020220140642 A


### Non-patent literature cited in the description


- UV Coatings: Basics, Recent Developments and New Application. Elsevier, 2007
- Principle of Polymerization. Wiley